# EUROPEAN PATENT SPECIFICATION

(11) **EP 3 746 532 B1**
(45) Date of publication and mention of the grant of the patent: **23.02.2022**
(21) Application number: 19730386.0
(22) Date of filing: 18.06.2019
(51) Int. Cl.: C11B 9/00, A23L 27/20, A23L 27/29, A23L 27/00, C07C 317/24, C07C 323/22, C07C 221/00, C07C 59/185, C07C 49/175

(54) **COMPOUNDS FOR PROVIDING A LONG-LASTING STRAWBERRY ODOR**
VERBINDUNGEN FÜR DIE BEREITSTELLUNG EINES LANGANHALTENDEN ERDBEERGERUCHS
COMPOSÉS POUR FOURNIR UNE ODEUR DE FRAISE DE LONGUE DURÉE

(30) Priority: 21.06.2018 EP 18179137
(43) Date of publication of application: 09.12.2020
(73) Proprietor: Firmenich SA, 1242 Satigny (CH)
(72) Inventor: HERRMANN, Andreas, 1211 Geneva 8 (CH); ETTER, Sylvain, 1211 Geneva 8 (CH); FESER, Johannes, 1211 Geneva 8 (CH); TRACHSEL, Alain, 1211 Geneva 8 (CH)
(74) Representative: Lavy, Séverine
(86) International application number: PCT/EP2019/066095
(87) International publication number: WO 2019/243369

(56) References cited:
- EP-A1- 2 046 147
- WO-A1-2016/116420
- WO-A2-2008/142591
- US-A1- 2013 202 746

## Description

### Technical field

The present invention relates to the field of perfumery. More particularly, it relates to compounds of formula (I) that are able to generate oct-2-en-4-one and thus to provide a long-lasting or substantive strawberry odor to the environment. Moreover, the present invention relates to a method of imparting a long-lasting strawberry odor to surfaces, such as hard surfaces, fabric, skin or hair. Furthermore, the present invention relates to the use of said compounds in perfumery, as well as the perfuming compositions or perfumed articles comprising the invention's compounds.

### Background of the Invention

Consumers often correlate the efficiency of perfumed articles with the long-lastingness or substantivity of perfume perception. Perfumes are composed of a multitude of different volatile compounds, which are applied to a surface from which they evaporate to be smelled. The perfume is applied to surfaces, such as hard surfaces, fabric, skin or hair, via a perfume composition or a perfumed consumer article, as for example fine fragrances or diverse washing and cleaning agents. Due to the high volatility of the fragrances, which constitute perfumes, the odor emitted from the perfumed surface can only be perceived over a limited amount of time. In particular the so-called top-notes of a perfume evaporate quite rapidly. They are the most volatile compounds of the composition and represent the freshness of a perfume. Top-notes typically comprise, among others, citrus, flowery, green and fruity notes, and especially the fruity notes are well-appreciated by the consumers. Several classes of fruity notes are used in perfumes, one example are notes resembling of red berries, such as raspberries or strawberries. Oct-2-ene-4-one can be used as a perfumery ingredient to provide a natural strawberry odor when used in a perfume composition or a perfumed consumer article. However, the compound is quite volatile, and its odor imparted to surfaces, such as hard surfaces, fabric, skin or hair, only lasts for a relatively short period of time. EP2046147 discloses a flavor precursor that can release a flavor compound, including oct-2-ene-4-one. The precursor disclosed in EP2046147 is added to food to slowly release the flavor upon heating and consumption.

Consumers seek for fragrances that are stable in the targeted application and at the same time long-lasting or substantive to be smelled for several hours or even days after application. In particular long-lasting red berry notes, such as e.g. strawberry notes, are desirable.

Therefore it is the goal of the present invention to provide a system that is able to deliver a long-lasting or substantive strawberry odor to the environment. Furthermore, another objective of the present invention is to find a method of imparting a long-lasting strawberry odor to surfaces, such as hard surfaces, fabric, skin or hair, via the application of perfuming compositions or perfumed articles.

### Description of the Invention

We have now found that some specific compounds derived from oct-2-ene-4-one can advantageously be employed to bring a long-lasting or substantive strawberry odor from a given surface into the environment, and thus to be useful as ingredients for perfuming compositions or perfumed articles.

Therefore, a first object of the present invention concerns a compound of formula 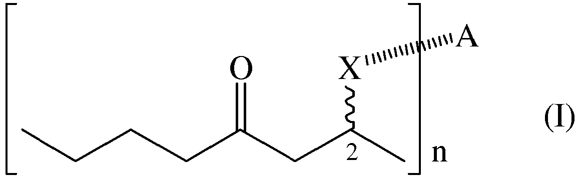 wherein
n represents an integer varying from 1 to 6;
A represents an n-valent C₂ to C₂₂ hydrocarbon group, optionally comprising one to eight ether groups and/or one or two functional groups selected from the group consisting of alcohol, ketone, aldehyde, ester, thioether, carboxylic acid, alkali carboxylate, amine, amide, carbamate, nitrile or thiol; and
X represents a group of formula (i) to (vi) 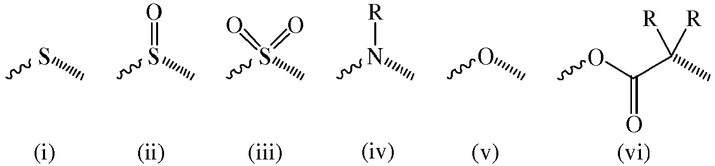
in which formulae the wavy line indicates the location of the bond between carbon 2 and X and the hatched line indicates the location of the bond between X and A; R representing independently of each other a hydrogen atom or a C₁ to C₄ linear or branched hydrocarbon group, provided that the hatched line is not directly linked to a heteroatom or to a carbonyl functional group of A, and provided that 2-(propylthio)octan-4-one, 4-oxooctan-2-yl 4-(benzyloxy)benzoate, 2-(methoxymethoxy)octan-4-one, 2-((2-methoxyethoxy)methoxy)octan-4-one, 2-(ethylamino)octan-4-one and 2-(benzylamino)octan-4-one are excluded.

If n > 1, then each X represents independent of each other either one of the groups of formula (i) to (vi). Preferably, if n > 1, each X represents the same group selected among formulae (i) to (vi).

It is understood that by "... hydrocarbon group ..." it is meant that said group consists of hydrogen and carbon atoms and can be in the form of an aliphatic hydrocarbon, i.e. linear or branched saturated hydrocarbon (e.g. alkyl group), a linear or branched unsaturated hydrocarbon (e.g. alkenyl or alkynyl group), a saturated cyclic hydrocarbon (e.g. cycloalkyl) or an unsaturated cyclic hydrocarbon (e.g. cycloalkenyl or cycloalkynyl), or can be in the form of an aromatic hydrocarbon, i.e. aryl group, or can also be in the form of a mixture of said type of groups, e.g. a specific group may comprise a linear alkyl, a branched alkenyl (e.g. having one or more carbon-carbon double bonds), a (poly)cycloalkyl and an aryl moiety, unless a specific limitation to only one type is mentioned. Similarly, in all the embodiments of the invention, when a group is mentioned as being in the form of more than one type of topology (e.g. linear, cyclic or branched) and/or being saturated or unsaturated (e.g. alkyl, aromatic or alkenyl), it is also meant a group which may comprise moieties having any one of said topologies or being saturated or unsaturated, as explained above. Similarly, in all the embodiments of the invention, when a group is mentioned as being in the form of one type of saturation or unsaturation, (e.g. alkyl), it is meant that said group can be in any type of topology (e.g. linear, cyclic or branched) or having several moieties with various topologies.

It is understood that with the terms "... a hydrocarbon group, optionally comprising ..." or "... a hydrocarbon group, optionally substituted with ..." it is meant that said hydrocarbon group optionally comprises alcohol, ketone, aldehyde, ether, thioether, ester, carboxylic acid, alkali carboxylate, amine, amide, carbamate, nitrile or thiol groups. These groups can either substitute a hydrogen atom of the hydrocarbon group and thus be laterally attached to said hydrocarbon, or substitute a carbon atom (if chemically possible) of the hydrocarbon group and thus be inserted into the hydrocarbon chain. For example, a -CH₂-CH₂-CHOH-CH₂- group represents a C₄ hydrocarbon group comprising an alcohol group (substitution of a hydrogen atom), a -CH₂-CH₂-COO-CH₂-CH₂-OCO-CH₂-CH₂- group represents a C₈ hydrocarbon group comprising two ester groups (substitution of carbon atoms/insertion into the hydrocarbon chain) and, similarly, a -CH₂-CH₂-O-CH₂-CH₂-O-CH₂-CH₂- group represents a C₆ hydrocarbon group comprising two ether groups. By contrast, the expression " ...alkyl or alkenyl group, optionally substituted with..." refers to an alkyl or alkenyl group wherein at least one hydrogen atom of the alkly or alkenyl group may be substituted by a functional group.

According to any embodiment of the invention, X represents preferably a group of formula (i) to (iv) or (vi), preferably a group of formula (i) to (iii) or (vi), even more preferably a group of formula (i) to (iii), most preferably a group of formula (i).

According to any embodiment of the invention, n is preferably an integer varying between 1 and 4, more preferably between 1 and 2, and even more preferably n may be 1.

According to any embodiment of the invention, A may represent an n-valent C₂ to C₂₂ hydrocarbon group, optionally comprising one to eight ether groups and/or with one or two functional groups selected from the group consisting of alcohol, ketone, aldehyde, ester, thioether, carboxylic acid, alkali carboxylate, amine, amide, carbamate, nitrile or thiol. Preferably, A may represent an n-valent C₂ to C₁₈ hydrocarbon group optionally comprising one to four ether groups and/or with one or two functional groups selected from the group consisting of alcohol, ketone, aldehyde, ester, carboxylic acid, amine, amide or carbamate. Preferably, A may represent an mono or di valent C₂ to C₁₈ hydrocarbon group, optionally comprising one to four ether groups and/or with one or two functional groups selected from the group consisting of alcohol, ketone, ester, carboxylic acid, amine, amide or carbamate. Preferably, A may represent a benzyl group or a mono or di valent C₂ to C₁₈ alkyl or alkenyl group, optionally substituted with one to four C₁₋₄ ether groups and/or with one or two functional groups selected from the group consisting of alcohol, ketone, C₁₋₁₀ ester, carboxylic acid, amine, C₁₋₄ amide or C₁₋₄ carbamate. Preferably, A may represent a benzyl group or a mono or di valent C₂ to C₁₈ alkyl or alkenyl group, optionally substituted with one or two functional groups selected from the group consisting of alcohol, ketone, C₁₋₄ ester, carboxylic acid, amine or C₁₋₄ carbamate. Preferably, A may represent a benzyl group or a mono valent C₄ to C₁₆ alkyl or alkenyl group or a mono valent C₂ to C₄ alkyl group substituted with one or two functional groups selected from the group consisting of alcohol, ketone, C₁₋₄ ester, carboxylic acid, amine or C₁₋₄ carbamate or A may represent a divalent C₂ to C₆ alkyl group substituted with one or two functional groups selected from the group consisting of alcohol, ketone, C₁₋₄ ester, carboxylic acid, amine or C₁₋₄ carbamate. Preferably, A may represent a benzyl group or a mono valent C₅ to C₁₅ alkyl or alkenyl group. Preferably, A may represent a mono valent C₆ to C₁₄ alkyl or alkenyl group. Even more preferably, A may represent a mono valent C₆ to C₁₄ linear alkyl or alkenyl group.

According to any one of the above embodiments, said compound of formula (I) is a 2-(alkylthio)octan-4-one, with the size of the alkyl group varying between C₆ and C₁₈, preferably between C₆ and C₁₄, such as 2-(octylthio)octan-4-one or 2-(dodecylthio)octan-4-one, a 2- or 3-((4-oxooctan-2-yl)thio)propanoic acid derivative, such as 2-((4-oxooctan-2-yl)thio)propanoic acid, 3-((4-oxooctan-2-yl)thio)propanoic acid or 2-methyl-3-((4-oxooctan-2-yl)thio)propanoic acid, a 2-((hydroxyalkyl)thio)octan-4-one derivative, with the size of the alkyl group varying between C₂ and C₁₈, preferably between C₂ and C₁₀, such as 2-((2-hydroxyethyl)thio)octan-4-one, an alkyl (2-((4-oxooctan-2-yl)thio)alkyl)carbamate derivative, with the size of the alkyl group varying between C₂ and C₁₈, preferably between C₂ and C₆, such as tert-butyl (2-((4-oxooctan-2-yl)thio)ethyl)carbamate, a 2-(alkylamino)octan-4-one, with the size of the alkyl group varying between C₆ and C₁₈, preferably between C₆ and C₁₄, such as 2-(dodecylamino)octan-4-one, a 2-((4-oxooctan-2-yl)amino)carboxylic acid derived from natural or unnatural amino acids, such as glycine, alanine, phenylalanine, arginine, histidine, lysine, aspartic acid, glutamic acid, cysteine, methionine, glutamine, asparagine, threonine, serine, leucine, isoleucine, valine, tyrosine or tryptophan, an alkyl 2-((4-oxooctan-2-yl)amino)carboxylate derived from natural or unnatural amino acids cited before, with the size of the alkyl group varying between C₁ and C₄, N,S-bis(4-oxooctan-2-yl)-L-cysteine, an alkyl N,S-bis(4-oxooctan-2-yl)cysteinate, with the size of the alkyl group varying between C₁ and C₄, such as methyl N,S-bis(4-oxooctan-2-yl)-L-cysteinate, a 2-(alkylsulfinyl)octan-4-one, with the size of the alkyl group varying between C₆ and C₁₈, preferably between C₆ and C₁₄, such as 2-(dodecylsulfinyl)octan-4-one, a 2-(alkylsulfonyl)octan-4-one, with the size of the alkyl group varying between C₆ and C₁₈, preferably between C₆ and C₁₄, such as 2-(dodecylsulfonyl)octan-4-one, a 4-oxooctan-2-yl alkanoate or alkenoate, with the size of the alkyl or alkenyl group varying between C₆ and C₁₈, preferably between C₆ and C₁₄, such as 4-oxooctan-2-yl undec-10-enoate, or a mono- or multi-(4-oxooctan-2-yl) aryl carboxylate, with the aryl carboxylate group being benzoic acid, phthalic acid, isophthalic acid, terephthalic acid or trimesic acid. Preferably, said compound of formula (I) is a 2-(alkylthio)octan-4-one, a 2-(alkylsulfinyl)octan-4-one, a 2-(alkylsulfonyl)octan-4-one or a 4-oxooctan-2-yl alkanoate or alkenoate, with the size of the respective alkyl or alkenyl group varying between C₆ and C₁₈, preferably between C₆ and C₁₄, such as 2-(octylthio)octan-4-one, 2-(dodecylthio)octan-4-one, 2-(dodecylsulfinyl)octan-4-one, 2-(dodecylsulfonyl)octan-4-one or 4-oxooctan-2-yl undec-10-enoate.

The compounds according to formula (I) are able to slowly generate oct-2-en-4-one over time and thus to provide a long-lasting strawberry odor to the environment. The compounds themselves are non-volatile and essentially odorless. At the same time they are relatively stable in perfuming compositions or perfumed articles. When exposed to a surface under environmental conditions, oct-2-en-4-one is believed to be formed by reaction with ambient humidity. The generation of oct-2-en-4-one may further be triggered by the presence of oxygen in the air, by pH changes, the presence of enzymes, or at increased temperature, or by other types of mechanisms, or by the combination of several mechanisms.

Non-volatile and essentially odorless compounds are advantageously characterized by a vapor pressure below 2.0 Pa, as obtained by calculation using the software EPIwin v. 3.10 (2000, available at the US Environmental Protection Agency). Preferably, said vapor pressure is below 0.2 Pa, or even more preferably below 0.02 Pa.

Oct-2-ene-4-one can be generated from the compounds of formula (I) in the (E) form, or in the (Z) form, or in mixtures thereof. The generation of (E)-oct-2-en-4-one or of mixtures of (E/Z)-oct-2-en-4-one with the (E)-isomer being the major component are preferred.

The compounds of formula (I) may be synthesized from oct-2-en-4-one by conventional methods. Preferably they are synthesized from (E)-oct-2-en-4-one or from mixtures of (E/Z)-oct-2-en-4-one with the (E)-isomer being the major component. Generally speaking, the invention's compounds are obtainable by the [1,4]-addition reaction between oct-2-en-4-one and a compound of formula [HX-]ₙ-A, wherein all the symbols have the meaning as indicated in formula (I).

Although it is not possible to provide an exhaustive list of the compounds of formula [HX-]ₙ-A which may be used in the synthesis of the invention's compounds, one can cite as preferred and non-limiting examples the following: hexane-1-thiol, octane-1-thiol, dodecane-1-thiol, hexadecane-1-thiol, octadecane-1-thiol, 2-mercaptoacetic acid, methyl or ethyl 2-mercaptoacetate, 2-mercaptopropanoic acid, methyl or ethyl 2-mercaptopropanoate, 3-mercaptopropanoic acid, methyl or ethyl 3-mercaptopropanoate, 3-mercapto-2-methylpropanoic acid, methyl or ethyl 3-mercapto-2-methylpropanoate, 2-mercaptoethanol, tert-butyl (2-mercaptoethyl)carbamate, 1-hexylamine, 1-octylamine, 1-decylamine, 1-dodecylamine, methyl or ethyl 2-amino-3-mercaptopropanoate or their corresponding hydrochlorides, natural or unnatural amino acids, such as glycine, alanine, phenylalanine, arginine, histidine, lysine, aspartic acid, glutamic acid, cysteine, methionine, glutamine, asparagine, threonine, serine, leucine, isoleucine, valine, tyrosine or tryptophan, as well as their corresponding methyl or ethyl esters.

Compounds of formula (I) can also be obtained by esterification of 2-hydroxyoctan-4-one by conventional methods. Preferably they are synthesized from 2-hydroxyoctan-4-one and an acid chloride of formula [ClCOCR₂]ₙ-A, wherein all the symbols have the meaning as indicated in formula (I).

Although it is not possible to provide an exhaustive list of the acid chlorides of formula [ClCOCR₂]ₙ-A which may be used in the synthesis of the invention's compounds, one can cite as preferred and non-limiting examples the acid chlorides of saturated, unsaturated or aromatic C₆ to C₁₈ carboxylic acids, such as: caprylic acid, capric acid, lauric acid, myristic acid, palmitic acid, stearic acid, octenoic acid, decenoic acid, decadienoic acid, undecenoic acid, lauroleic acid, laurolinoleic acid, 4-hexadecenoic acid, oleic acid, linoleic acid, adipic acid, pimelic acid, sebacic acid, dodecanedioic acid, propane-1,2-3-tricarboxylic acid, aconitic acid, benzoic acid, phthalic acid, isophthalic acid, terephthalic acid or trimesic acid.

According to any embodiment, the compound of formula (I) is encapsulated. At least one compound of formula (I) can be encapsulated in a microcapsule. In one embodiment, at least one compound of formula (I) is encapsulated in a core-shell microcapsule wherein the compound of formula (I) is contained in the core surrounded by the shell. The shell of the microcapsule protects the compound of formula (I) from the environment. The shell is made of material which is able to release the compound of formula (I). In one embodiment, the shell is made of material which is able to release the compound of formula (I) upon breakage of the shell and/or by diffusion through the shell. A person skilled in the art is well aware of processes to prepare said microcapsules.

The nature of the polymeric shell from the microcapsules of the invention can vary. As non-limiting examples, the shell can be aminoplast-based, polyurea-based or polyurethane-based. The shell can also be hybrid, namely organic-inorganic such as a hybrid shell composed of at least two types of inorganic particles that are cross-linked, or yet a shell resulting from the hydrolysis and condensation reaction of a polyalkoxysilane macro-monomeric composition.

According to an embodiment, the shell comprises an aminoplast copolymer, such as melamine-formaldehyde or urea-formaldehyde or cross-linked melamine formaldehyde or melamine glyoxal.

According to another embodiment the shell is polyurea-based made from, for example but not limited to isocyanate-based monomers and amine-containing crosslinkers such as guanidine carbonate and/or guanazole. Preferred polyurea microcapsules comprise a polyurea wall which is the reaction product of the polymerisation between at least one polyisocyanate comprising at least two isocyanate functional groups and at least one reactant selected from the group consisting of an amine (for example a water soluble guanidine salt and guanidine); a colloidal stabilizer or emulsifier; and an encapsulated perfume. However, the use of an amine can be omitted.

According to a particular embodiment the colloidal stabilizer includes an aqueous solution of between 0.1% and 0.4% of polyvinyl alcohol, between 0.6% and 1% of a cationic copolymer of vinylpyrrolidone and of a quaternized vinylimidazol (all percentages being defined by weight relative to the total weight of the colloidal stabilizer). According to another embodiment, the emulsifier is an anionic or amphiphilic biopolymer preferably chosen from the group consisting of gum Arabic, soy protein, gelatin, sodium caseinate and mixtures thereof.

According to another embodiment, the shell is polyurethane-based made from, for example but not limited to polyisocyanate and polyols, polyamide, polyester, etc.

The preparation of an aqueous dispersion/slurry of core-shell microcapsules is well known by a skilled person in the art. In one aspect, said microcapsule wall material may comprise any suitable resin and especially including melamine, glyoxal, polyurea, polyurethane, polyamide, polyester, etc. Suitable resins include the reaction product of an aldehyde and an amine, suitable aldehydes include, formaldehyde and glyoxal. Suitable amines include melamine, urea, benzoguanamine, glycoluril, and mixtures thereof. Suitable melamines include, methylol melamine, methylated methylol melamine, imino melamine and mixtures thereof. Suitable ureas include, dimethylol urea, methylated dimethylol urea, urea-resorcinol, and mixtures thereof. Suitable materials for making may be obtained from one or more of the following companies Solutia Inc. (St Louis, Missouri U.S.A.), Cytec Industries (West Paterson, New Jersey U.S.A.), Sigma-Aldrich (St. Louis, Missouri U.S.A.).

According to a particular embodiment, the core-shell microcapsule is a formaldehyde-free capsule. A typical process for the preparation of aminoplast formaldehyde-free microcapsules slurry comprises the steps of 1) preparing an oligomeric composition comprising the reaction product of, or obtainable by reacting together
a) a polyamine component in the form of melamine or of a mixture of melamine and at least one C₁-C₄ compound comprising two NH₂ functional groups;
b) an aldehyde component in the form of a mixture of glyoxal, a C₄-₆ 2,2-dialkoxy-ethanal and optionally a glyoxalate, said mixture having a molar ratio glyoxal/C₄-₆ 2,2-dialkoxy-ethanal comprised between 1/1 and10/1; and
c) a protic acid catalyst;

2) preparing an oil-in-water dispersion, wherein the droplet size is comprised between 1 and 600 um, and comprising:
   i. an oil;
   ii. a water medium
   iii. at least an oligomeric composition as obtained in step 1;
   iv. at least a cross-linker selected amongst

   A) C₄-C₁₂ aromatic or aliphatic di- or tri-isocyanates and their biurets, triurets, trimmers, trimethylol propane-adduct and mixtures thereof; and/or
   B) a di- or tri-oxiran compounds of formula A-(oxiran-2-ylmethyl)ₙ
      wherein n stands for 2 or 3 and 1 represents a C₂-C₆ group optionally comprising from 2 to 6 nitrogen and/or oxygen atoms;
      v. optionally a C₁-C₄ compounds comprising two NH₂ functional groups;
3) Heating said dispersion;
4) Cooling said dispersion.

This process is described in more details in WO 2013/068255, the content of which is included by reference.

According to another embodiment, the shell of the microcapsule is polyurea-or polyurethane-based. Examples of processes for the preparation of polyurea and polyureathane-based microcapsule slurry are for instance described in WO2007/004166, EP 2300146, EP2579976 the contents of which is also included by reference. Typically a process for the preparation of polyurea or polyurethane-based microcapsule slurry include the following steps:
a) Dissolving at least one polyisocyanate having at least two isocyanate groups in an oil to form an oil phase;
b) Preparing an aqueous solution of an emulsifier or colloidal stabilizer to form a water phase;
c) Adding the oil phase to the water phase to form an oil-in-water dispersion, wherein the mean droplet size is comprised between 1 and 500 µm, preferably between 5 and 50 µm;
d) Applying conditions sufficient to induce interfacial polymerisation and form microcapsules in form of a slurry.

Examples of microcapsules suitable for use in the present invention include, but are not limited to the microcapsules disclosed in International Patent Application Publication No. WO 2007/026307 A2. Further examples include the microcapsules disclosed in International Patent Application Publication No. WO 2014/029695 A1. Additional examples include the microcapsules disclosed in International Patent Application Publication No. WO 2006/006003 A1. Additional examples include the microcapsules disclosed in International Patent Application Publication No. WO 2006/018964 A1. Additional examples include the microcapsules disclosed in International Patent Application Publication No. WO 2007/096790 A1. Additional examples include the microcapsules disclosed in International Patent Application Publication No. WO 2009/153695 A1. Additional examples include the microcapsules disclosed in European Patent No. EP2379047.

Examples of methods to encapsulate the compound of formula (I) include, but are not limited to the microcapsules disclosed in International Patent Application Publication No. WO 2007/026307 A2. Further examples include the microcapsules disclosed in International Patent Application Publication No. WO 2014/029695 A1. Additional examples include the microcapsules disclosed in International Patent Application Publication No. WO 2006/006003 A1. Additional examples include the microcapsules disclosed in International Patent Application Publication No. WO 2006/018964 A1. Additional examples include the microcapsules disclosed in International Patent Application Publication No. WO 2007/096790 A1. Additional examples include the microcapsules disclosed in International Patent Application Publication No. WO 2009/153695 A1. Additional examples include the microcapsules disclosed in European Patent No. EP2379047.

As mentioned above, the invention concerns the use of a compound of formula (I) as perfuming ingredient to provide a long-lasting strawberry odor to the environment. In other words, it concerns a method or a process to confer, enhance, improve or modify the strawberry odor properties of a perfuming composition or of a perfumed article or of a surface, which method comprises adding to said composition or article an effective amount of at least a compound of formula (I). By "use of a compound of formula (I)" it has to be understood here also the use of any composition containing a compound of formula (I) and which can be advantageously employed in the perfumery industry.

By "perfuming ingredient" it is meant here a compound, which is used in a perfuming preparation or a composition to impart a hedonic effect. In other words such perfuming ingredient, to be considered as being a perfuming one, must be recognized by a person skilled in the art as being able to impart or modify in a positive or pleasant way the odor of a composition, and not just as having an odor.

The expression "strawberry odor" or "strawberry note" should be understood as an odor reminding that of a strawberry fruit, for examples, compounds described as having a strawberry odor are oct-2-en-4-one, 2,5-dimethyl-4-hydroxy-2H-furan-3-one (Furaneol^{®}), 5-ethyl-4-hydroxy-2-methyl-3(2H)-furanone, 2,5-dimethyl-4-oxo-4,5-dihydrofuran-3-yl acetate, ethyl 3-methyl-3-phenyloxirane-2-carboxylate (Strawberry Pure^{®}), ethyl 3-phenyloxirane-2-carboxylate, methyl 2-acetamidobenzoate or 3-phenylpropyl 3-methylbutanoate, 2-methyl-4-oxo-4H-pyran-3-yl propionate, 2-methylpent-2-enoic acid or (2S,5S)-2-(tert-butyl)-5-methyl-2-propyltetrahydrofuran (Dihydrocassyrane^{®}).

For sake of clarity, a long-lasting effect is typically achieved if, after a certain time, e.g. after several hours or days, a given compound emits higher amounts of an odor into the environment than a reference compound providing the same type of odor. Thus, the expression "long-lasting strawberry odor" when referring to the compound of formula (I) of the invention should be understood as an increase of duration of the strawberry odor perception (release of oct-2-ene-4-one in the atmosphere) as compared to the one of the oct-2-ene-4-one molecule alone, and measured under the same conditions for example after several hours (6 or 8 hours) or days (1 or 3 days).

Said compositions, which in fact can be advantageously employed as perfuming ingredients, are also an object of the present invention.

Therefore, another object of the present invention is a flavoring or perfuming composition comprising:
i) as flavoring or perfuming ingredient, at least one invention's compound as defined above;
ii) at least one ingredient selected from the group consisting of a flavor or perfumery carrier and a flavor or perfumery base; and
iii) optionally at least one flavor or perfumery adjuvant.

Preferably, the invention composition is a flavoring composition.

By "perfumery carrier" it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, benzyl benzoate, benzyl alcohol, 2-(2-ethoxyethoxy)-1-ethano, tri-ethyl citrate or mixtures thereof, which are the most commonly used. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

Solid carrier is meant to designate a material to which the perfuming composition or some element of the perfuming composition can be chemically or physically bound. In general such solid carriers are employed either to stabilize the composition, or to control the rate of evaporation of the compositions or of some ingredients. The use of solid carrier is of current use in the art and a person skilled in the art knows how to reach the desired effect. However by way of non-limiting example of solid carriers, one may cite absorbing gums or polymers or inorganic material, such as porous polymers, cyclodextrins, wood based materials, organic or inorganic gels, clays, gypsum talc or zeolites.

As other non-limiting examples of solid carriers, one may cite encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation technique.

As non-limiting examples of solid carriers, one may cite in particular the core-shell capsules with resins of aminoplast, polyamide, polyester, polyurea or polyurethane type or a mixture threof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycoluryl, melamine, methylol melamine, methylated methylol melamine, guanazole and the like, as well as mixtures thereof. Alternatively one may use preformed resins alkylolated polyamines such as those commercially available under the trademark Urac^{®} (origin: Cytec Technology Corp.), Cy mel^{®} (origin: Cytec Technology Corp.), Urecoll^{®} or Luracoll^{®} (origin: BASF).

Other resins are those produced by the polycondensation of an a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate.

Some of the seminal literature related to the encapsulation of perfumes by polycondensation of amino resins, namely melamine based resins with aldehydes includes represented by articles such as those published by K. Dietrich et al. Acta Polymerica, 1989, vol. 40, pages 243, 325 and 683, as well as 1990, vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinency, which disclose suitable uses of such microcapsules, are represented for example by the article of H.Y.Lee et al. Journal of Microencapsulation, 2002, vol. 19, pages 559-569, international patent publication WO 01/41915 or yet the article of S. Bône et al. Chimia, 2011, vol. 65, pages 177-181.

By "perfumery base" we mean here a composition comprising at least one perfuming co-ingredient.

Said perfuming co-ingredient is not of formula (I). Moreover, by "perfuming co-ingredient" it is meant here a compound, which is used in a perfuming preparation or a composition in addition to the perfuming ingredient of formula (1), and imparting such as the perfuming ingredient of formula (I) a hedonic effect. In other words such a co-ingredient, to be considered as being a perfuming one, must be recognized by a person skilled in the art as being able to impart or modify in a positive or pleasant way the odor of a composition, and not just as having an odor.

In particular, perfuming co-ingredients that might be used in a perfuming preparation or a composition according to the invention include as non-limiting examples:
a) natural or nature-identical ingredients and natural extracts, preferably those obtained from red berries, such as strawberries or raspberries;
b) ingredients with strawberry notes, such as oct-2-en-4-one, 2,5-dimethyl-4-hydroxy-2*H*-furan-3-one (Furaneol^{®}), 5-ethyl-4-hydroxy-2-methyl-3(2*H*)-furanone, 2,5-dimethyl-4-oxo-4,5-dihydrofuran-3-yl acetate, ethyl 3-methyl-3-phenyloxirane-2-carboxylate (Strawberry Pure^{®}), ethyl 3-phenyloxirane-2-carboxylate, methyl 2-acetamidobenzoate or 3-phenylpropyl 3-methylbutanoate, 2-methyl-4-oxo-4*H*-pyran-3-yl propionate, 2-methylpent-2-enoic acid or (2S,5S)-2-(*tert*-butyl)-5-methyl-2-propyltetrahydrofuran (Dihydrocassyrane^{®});
c) ingredients with raspberry notes, such as 4-(4-methoxyphenyl)butan-2-one (raspberry ketone);
d) ingredients with other fruity notes, such as ethyl butyrate, 5-heptyldihydrofuran-2(3*H*)-one, ethyl 2-(2-methyl-1,3-dioxolan-2-yl)acetate (Fructone^{®}) or diethyl cyclohexane-1,4-dicarboxylate (Fructalate^{®});
e) ingredients with caramel notes, such as 2-ethyl-3-hydroxy-4*H*-pyran-4-one;
f) ingredients with citrus notes, such as 1-methyl-4-(1-methylethenyl)-cyclohexene (Limonene);
g) ingredients with flowery citrus notes, such as methyl 2-(-3-oxo-2-pentylcyclopentyl)acetate (Hedione^{®});
h) ingredients with flowery notes, such as 3,7-dimethylocta-1,6-dien-3-ol (Linalool), 3-methylbutyl 2-hydroxybenzoate, hexyl 2-hydroxybenzoate, benzyl 2-hydroxybenzoate or cyclohexyl 2-hydroxybenzoate;
i) ingredients with flowery woody notes, such as (E)-4-(2,6,6-trimethylcyclohex-1- or 2-en-1-yl)but-3-en-2-one (beta- or alpha-Ionone), (E)-4-(2,2-dimethyl-6-methylenecyclohexyl)but-3-en-2-one (gamma-Ionone) or 1-(octahydro-2,3,8,8-tetrame-2-naphthalenyl)-1-ethanone (Iso E Super^{®}, mixture of isomers);
j) ingredients with green notes, such as (Z)-hex-3-en-1-ol, (Z)-hex-3-en-1-yl acetate, (Z)-hex-3-en-1-yl butyrate, (Z)-hex-3-en-1-yl benzoate, (Z)-hex-3-en-1-yl 2-hydroxybenzoate, 1-(3,3- or 5,5-dimethylcyclohex-1-en-1-yl)pent-4-en-1-one (Neobutenone^{®});
k) ingredients with musky notes, such as oxacyclohexadecan-2-one (Exaltolide^{®}), oxacyclohexadec-12- and/or 13-en-2-one (Habanolide^{®}), 3-methylcyclopentadecan-1-one (Muscone^{®}), (Z)-3-methylcyclopentadec-5-en-1-one (Muscenone^{®}) or 2-(1-(3,3-dimethylcyclohexyl)ethoxy)-2-methylpropyl propionate (Helvetolide^{®}).

Furthermore, the person skilled in the art is able to select other perfuming co-ingredients on the basis of the general knowledge and according to the intended use or application and the desired organoleptic effect to be achieved. The nature and type of these other perfuming co-ingredients do not warrant a more detailed description here, which in any case would not be exhaustive. In general terms, these perfuming co-ingredients belong to chemical classes as varied as alcohols, lactones, aldehydes, ketones, esters, ethers, acetates, nitriles, terpenoids, nitrogenous or sulfurous heterocyclic compounds and essential oils, and said perfuming co-ingredients can be of natural or synthetic origin. A perfumery base according to the invention may not be limited to the above mentioned perfuming co-ingredients, and many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds, or can be an encapsulated perfume.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well-known to a person skilled in the art. However, one may cite as specific non-limiting examples the following: viscosity agents (e.g. surfactants, thickeners, gelling and/or rheology modifiers), stabilizing agents (e.g. preservatives, antioxidants, heat/light and or buffers or chelating agents, such as BHT), coloring agents (e.g. dyes and/or pigments), preservatives (e.g. antibacterial or antimicrobial or antifungal or anti-irritant agents), abrasives, skin cooling agents, fixatives, insect repellants, ointments, vitamins and mixture thereof.

It is understood that a person skilled in the art is perfectly able to design optimal formulations for the desired effect by admixing the above mentioned components of a perfuming composition, simply by applying the standard knowledge of the art as well as by trial and error methodologies.

Other suitable perfumery adjuvants optionally used in combination with the compounds according to the present invention comprise tertiary amines, in particular those with high water solubility, such as triethanolamine, methyldiethanolamine, dimethylethanolamine, alkyldiethanolamines and ethoxylated alkyldiethanolamines.

A particular aspect of the invention's perfumery compositions concerns the ones further comprising (in addition to the above mentioned compositions) at least one compound selected amongst the isothiazolones of formula 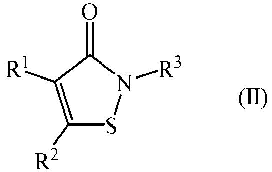 wherein
R¹ and R² represent, separately and independently of each other, a hydrogen atom, a halogen atom, preferably chlorine, a C₁-C₄ linear or branched alkyl group, an amino group or a benzylamino group; or, alternatively, R¹ and R² are taken together to represent a phenyl or pyridine ring, possibly substituted with one to four C₁-C₄ linear or branched alkyl or alkenyl groups and/or one to two halogen atoms, preferably chlorine atoms; and
R³ represents a hydrogen atom, an alkali metal atom, in particular Na or K, a phenyl or benzyl group possibly substituted with one or two halogen atoms and/or one or two methyl, trifluoromethyl, methoxy or amino groups, an amine group, or a C₁-C₈ unsaturated, linear, branched or cyclic hydrocarbon group possibly substituted with one or two nitrogen, oxygen or halogen atoms.

According to a particular embodiment of the invention said compound of formula (II) is one wherein R¹ and R² represent, separately and independently of each other, a hydrogen atom, a chlorine atom or a methyl group or, alternatively, R¹ and R² are taken together to represent a phenyl ring, and R³ represents a hydrogen atom or a methyl group.

According to a particular embodiment of the invention, said compound of formula (II) is selected from the group of isothiazolones consisting of 1,2-benzisothiazol-3(2*H*)-one, 4- or 5-chloro-2-methylisothiazol-3(2*H*)-one or 2-methylisothiazol-3(2*H*)-one, or more preferably 5-chloro-2-methylisothiazol-3(2*H*)-one or 1,2-benzisothiazol-3(2*H*)-one, and most preferably 1,2-benzisothiazol-3(2*H*)-one.

An invention's composition consisting of at least one compound of formula (I) and at least one perfumery carrier represents a particular embodiment of the invention as well as a perfuming composition comprising at least one compound of formula (I), at least one perfumery carrier, at least one perfumery base, and optionally at least one perfumery adjuvant.

According to a particular embodiment, the compositions mentioned above, may comprise more than one compound of formula (I) or may comprise, in addition to a compound of formula (I), other compounds of similar or different nature being able to generate other fragrances than oct-2-en4-one and enable the perfumer to prepare accords or perfumes possessing the odor tonality of various compounds of the invention, creating thus new building block for creation purposes.

For the sake of clarity, it is also understood that any mixture resulting directly from a chemical synthesis, e.g. a reaction medium without an adequate purification, in which the compound of the invention would be involved as a starting, intermediate or endproduct could not be considered as a perfuming composition according to the invention as far as said mixture does not provide the inventive compound in a suitable form for perfumery. Thus, unpurified reaction mixtures are generally excluded from the present invention unless otherwise specified.

Furthermore, the invention's compound of formula (I) can also be advantageously used in all the fields of flavor or modern perfumery, i.e. fine or functional perfumery, to positively impart a long-lasting or substantive strawberry odor to a consumer product into which said compound (I) is added.

Consequently, a perfumed consumer product which comprises:
i) as perfuming ingredient, at least one compound of formula (I) or a perfuming composition, as defined above; and
ii) a perfumery consumer base;
is also an object of the present invention.

The invention's compound can be added as such or as part of an invention's perfuming composition.

For the sake of clarity, "perfumed consumer product" is meant to designate a consumer product which delivers at least a pleasant perfuming effect to the surface or space to which it is applied (e.g. skin, hair, textile, or home surface). In other words, a perfumed consumer product according to the invention is a perfumed consumer product which comprises a functional formulation, as well as optionally additional benefit agents, corresponding to the desired consumer product, and an olfactive effective amount of at least one invention's compound. For the sake of clarity, said perfumed consumer product is a non-edible product.

The nature and type of the constituents of the perfumed consumer product do not warrant a more detailed description here, which in any case would not be exhaustive, the skilled person being able to select them on the basis of his general knowledge and according to the nature and the desired effect of said product.

Non-limiting examples of suitable perfumed consumer products include a perfume, such as a fine perfume, a splash or eau de parfum, a cologne or a shave or after-shave lotion; a fabric care product, such as a liquid or solid detergent, a fabric softener, a liquid or solid scent booster, a fabric refresher, an ironing water, a paper, a bleach, a carpet cleaner, a curtain-care product; a body-care product, such as a hair care product (e.g. a shampoo, a coloring preparation or a hair spray, a color-care product, a hair shaping product, a dental care product), a disinfectant, an intimate care product; a cosmetic preparation (e.g. a skin cream or lotion, a vanishing cream or a deodorant or antiperspirant (e.g. a spray or roll on), a hair remover, a tanning or sun or after sun product, a nail product, a skin cleansing, a makeup); or a skin-care product (e.g. a soap, a shower or bath mousse, oil or gel, or a hygiene product or a foot/hand care products); an air care product, such as an air freshener or a "ready to use" powdered air freshener which can be used in the home space (rooms, refrigerators, cupboards, shoes or car) and/or in a public space (halls, hotels, malls, etc..); or a home care product, such as a mold remover, a furnisher care product, a wipe, a dish detergent or a hard-surface (e.g. a floor, bath, sanitary or a window-cleaning) detergent; a leather care product; a car care product, such as a polish, a wax or a plastic cleaner.

Preferred perfuming compositions or perfumed articles are perfumes, fabric or hard-surface detergents, hair-care products and fabric softeners or refreshers.

Typical examples of fabric detergents or softener compositions into which the compounds of the invention can be incorporated are described in WO 97/34986, or in US patents 4,137,180 and 5,236,615 or in EP 799 885. Other typical detergents and softening compositions which can be used are described in works such as Ullmann's Encyclopedia of Industrial Chemistry, vol. 20, Wiley-VCH, Weinheim, p. 355-540 (2012); Flick, Advanced Cleaning Product Formulations, Noye Publication, Park Ridge, New Jersey (1989); Showell, in Surfactant Science Series, vol. 71: Powdered Detergents, Marcel Dekker, New York (1988); Proceedings of the World Conference on Detergents (4th, 1998, Montreux, Switzerland), AOCS print.

Some of the above-mentioned consumer product bases may represent an aggressive medium for the invention's compound, so that it may be necessary to protect the latter from premature decomposition, for example by encapsulation.

The proportions in which the compounds according to the invention can be incorporated into the various aforementioned articles or compositions vary within a wide range of values. These values are dependent on the nature of the article to be perfumed and on the desired organoleptic effect as well as the nature of the co-ingredients in a given base when the compounds according to the invention are mixed with perfuming co-ingredients, solvents or additives commonly used in the art.

For example, in the case of perfuming compositions, typical concentrations are in the order of 0.0001 % to 10 % by weight, or even more, of the compounds of the invention based on the weight of the composition into which they are incorporated, preferably, . 0.001 % to 5 % by weight, of the compounds of the invention based on the weight of the composition into which they are incorporated, preferably, . 0.001 % to 3 % by weight, of the compounds of the invention based on the weight of the composition into which they are incorporated, even more preferably, 0.005 % to 1 % by weight, of the compounds of the invention based on the weight of the composition into which they are incorporated. In the case of perfumed consumer products, typical concentrations are in the order of 0.01 % to 5 % by weight, or even more, of the compounds of the invention based on the weight of the consumer product into which they are incorporated.

As mentioned above, the invention concerns a method of imparting a long-lasting or substantive strawberry odor to surfaces, such as hard surfaces, fabric, skin or hair. Perfume ingredients that provide a strawberry odor to the environment by evaporation from a surface are typically not very long-lasting or substantive. As outlined above, one reason for this is their relatively high volatility, which guarantees an efficient evaporation after surface deposition. Another reason for this is that quite often only small amounts of these compounds are efficiently deposited on the surface. This is in particular the case if they are applied to a surface via perfuming compositions or perfumed articles, which are rinsed after application. This rinsing step also carries away a large amount of the perfume, which is supposed to remain on the target surface. Examples for this case are washing and cleaning agents, such as hard surface cleaners, detergents, shower gels shampoos and the like, which are rinsed off after application. Furthermore, perfuming a surface by bringing it into contact with perfuming compositions or perfumed articles from which the perfume is deposited onto the surface by a partition equilibrium between the perfuming compositions or perfumed articles and the corresponding surface might be inefficient for the deposition of the perfume. Examples for this case are conditioners or surface refreshers, such as fragrance softeners, which are brought into contact with the target and then removed or left drying. Compounds of formula (I) according to the present invention are suitable to enhance the deposition of the perfume and in particular of oct-2-en-4-one and thus to impart a long-lasting strawberry odor to surfaces, such as hard surfaces, fabric, skin or hair.

Therefore, another aspect of the present invention concerns a method of imparting a long-lasting or substantive strawberry odor to surfaces, such as hard surfaces, fabric, skin or hair, by adding at least one compound of formula (I) to perfuming compositions or perfumed articles and applying them to the corresponding targeted surface.

### Examples

The invention is hereafter described in a more detailed manner by way of the following examples, wherein the abbreviations have the usual meaning in the art, temperatures are indicated in degrees centigrade (°C). NMR spectral data were recorded on a Bruker AMX 500 spectrometer in CDCl₃ at 500 MHz for ¹H and at 125.8 MHz for ¹³C if not indicated otherwise, the chemical displacements d are indicated in ppm with respect to Si(CH₃)₄ as the standard, the coupling constants J are expressed in Hz (br. = broad peak). Reactions were carried out in standard glassware under N₂. Commercially available reagents and solvents were used without further purification if not stated otherwise.

Although specific conformations or configurations are indicated for some of the compounds, this is not meant to limit the use of these compounds to the isomers described. According to the invention, all possible conformation or configuration isomers are expected to have a similar effect.

### Example 1

### Preparation of compounds according to formula (I) providing a long-lasting strawberry odor

(a) *Synthesis of (±)-2-(octylthio)octan-4-one* **(Compound 1)**
   (E)-2-Octen-4-one (2.00 g, 15.9 mmol) and 1-octanethiol (2.27 g, 15.5 mmol) were stirred at room temperature for 2 weeks. The remaining volatiles were removed by bulb-to-bulb distillation to give 3.86 g (89%) of a slightly yellow oil.
   ¹H-NMR: 3.29-3.21 (m, 1 H), 2.70 (dd, J = 16.7, 6.1 Hz, 1 H), 2.52 (dd, J = 16.7, 8.0 Hz, 1 H), 2.52 (t, J = 7.4 Hz, 2 H), 2.47-2.35 (m, 2 H), 1.62-1.52 (m, 4 H), 1.42-1.20 (m, 15 H), 0.91 (t, J = 7.4 Hz, 3 H), 0.88 (t, J = 6.7, 3 H).
   ¹³C-NMR: 209.16, 50.14, 43.40, 35.11, 31.83, 30.88, 29.74, 29.21, 29.20, 29.02, 25.77, 22.66, 22.32, 21.73, 14.10, 13.86.
(b) *Synthesis of (±)-2-(dodecylthio)octan-4-one* **(Compound 2)**
   (E)-2-Octen-4-one 1.00 g, 7.9 mmol) and 1-dodecanethiol (1.57 g, 7.8 mmol) were stirred at room temperature for 2 weeks. Column chromatography (SiO₂, *n*-heptane then n-heptane/ethyl acetate 99:1 and 97:3) gave 1.21 g (46%) of a yellow oil.
   ¹H-NMR: 3.29-3.21 (m, 1 H), 2.70 (dd, J = 16.7, 5.8 Hz, 1 H), 2.52 (dd, J = 16.3, 8.0 Hz, 1 H), 2.52 (t, J = 7.5 Hz, 2 H), 2.47-2.35 (m, 2 H), 1.61-1.52 (m, 4 H), 1.40-1.20 (m, 23 H), 0.91 (t, J = 7.1 Hz, 3 H), 0.88 (t, J = 7.1 Hz, 3 H).
   ¹³C-NMR: 209.16, 50.15, 43.40, 35.11, 31.93, 30.88, 29.74, 29.67, 29.65, 29.62, 29.55, 29.37, 29.26, 29.03, 25.77, 22.70, 22.32, 21.73, 14.13, 13.86.
(c) *Synthesis of (±)-2-((4-oxooctan-2-yl)thio)propanoic acid* **(Compound 3)**
   (E)-2-Octen-4-one (4.00 g, 31.7 mmol) and 2-mercaptopropanoic acid (thiolactic acid, 3.30 g, 31.1 mmol) were stirred at room temperature for 23 h. After dilution with *tert-*BuOMe, the mixture washed with water and a saturated aqueous solution of NaCl, dried (Na₂SO₄) and concentrated under vacuum (at a maximum temperature of 45°C) to give the crude compound as a yellow oil. Flash chromatography (SiO₂, n-heptane/tert-BuOMe 9:1 to 1:1) gave 4.99 g (68%) of a yellow oil, as a mixture of diastereoisomers (ca. 1.5:1).
   ¹H-NMR (major isomer): 10.29 (br. s, 1 H), 3.58-3.44 (m, 2 H), 2.82 (dd, J = 17.0, 6.1 Hz, 1 H), 2.57 (dd, J = 17.0, 7.4 Hz, 1 H), 2.47-2.35 (m, 2H), 1.61-1.51 (m, 2 H), 1.44 (d, J = 7.0 Hz, 3 H), 1.36-1.25 (m, 2 H), 1.30 (d, J = 7.0 Hz, 3 H), 0.91 (t, J = 7.4 Hz, 3 H).
   ¹³C-NMR (major isomer): 209.14, 179.46, 50.15, 43.28, 40.83, 36.13, 25.74, 22.28, 21.71, 17.34, 13.84.
(d) *Synthesis of (±)-3-((4-oxooctan-2-yl)thio)propanoic acid* **(Compound 4)**
   As described for Compound 3 with (E)-2-octen-4-one (2.00 g, 15.9 mmol) and 3-mercaptopropanoic acid (1.65 g, 15.6 mmol). Flash chromatography (SiO₂, n-heptane/tert-BuOMe 9:1 to 7:3) gave 0.39 g (11%) of a yellow oil.
   ¹H-NMR (400 MHz): 8.24 (br. s, 1 H), 3.34-3.22 (m, 1 H), 2.84-2.76 (m, 2 H), 2.72 (dd, J = 17.0, 5.9 Hz, 1 H), 2.70-2.63 (m, 2 H), 2.55 (dd, J = 17.0, 7.7 Hz, 1 H), 2.41 (dt, J = 7.4, 2.1 Hz, 2 H), 1.62-1.51 (m, 2 H), 1.37-1.24 (m, 2 H), 1.29 (d, J = 6.7 Hz, 3 H), 0.91 (t, J = 7.3 Hz, 3 H).
   ¹³C-NMR (100.6 MHz): 209.09, 177.59, 49.93, 43.37, 35.29, 34.66, 25.74, 25.41, 22.29, 21.70, 13.84.
(e) *Synthesis of (±)-2-methyl-3-((4-oxooctan-2-yl)thio)propanoic acid* **(Compound 5)**
   As described for Compound 3 with (E)-2-octen-4-one (2.00 g, 15.9 mmol) and 3-mercapto-2-methylpropanoic acid for 45 h. Bulb-to-bulb distillation (100°C, 0.4 mbar) to remove remaining 2-octen-4-one and flash chromatography (SiO₂, n-heptane/tert-BuOMe 9:1 to 1:1) gave 2.47 g (63%) of a yellow oil as a mixture of diastereoisomers (ca. 1:1).
   ¹H-NMR: 9.26 (br. s, 1 H), 3.32-3.22 (m, 1 H), 2.92-2.85 (m, 1 H), 2.77-2.65 (m, 2 H), 2.64-2.50 (m, 2 H), 2.47-2.35 (m, 2 H), 1.62-1.51 (m, 2 H), 1.37-1.24 (m, 8 H), 0.91 (t, J = 7.4 Hz, 3 H).
   ¹³C-NMR: 209.13 and 209.10, 181.11 and 181.02, 50.01 and 50.00, 43.39 and 43.35, 40.25 and 40.13, 35.79 and 35.65, 33.81 and 33.77, 25.74, 22.29, 21.77 and 21.71, 16.73 and 16.61, 13.85 (q).
(f) *Synthesis of (±)-2-((2-hydroxyethyl)thio)octan-4-one* **(Compound 6)**
   A solution of 2-mercaptoethanol (3.12 g, 39.5 mmol) in tetrahydrofuran (THF, 25 mL) was added dropwise to a solution of (E)-2-octen-4-one (5.37 g, 41.6 mmol) and 1,8-diazabicyclo[5.4.0]undec-7-ene (DBU, 0.65 mL, 4.3 mmol) in THF (50 mL) at 45-50°C. After stirring at ca. 50°C overnight, the reaction mixture was treated with a mixture of ethyl acetate and an aqueous solution of NaHCO₃ (10%). After decanting, the organic layer was washed with a saturated aqueous solution of NaCl and the aqueous layer re-extracted with ethyl acetate. The organic phases were dried (Na₂SO₄) and concentrated. Bulb-to-bulb distillation (150°C, 0.056 mbar) gave 7.66 g (90%) of a colorless oil.
   ¹H-NMR: 3.82-3.70 (m, 2 H), 3.36-3.27 (m, 1 H), 2.83-2.68 (m, 4 H), 2.56 (dd, J = 17.0, 7.1 Hz, 1 H), 2.47-2.36 (m, 2 H), 1.61-1.52 (m, 2 H), 1.37-1.27 (m, 2 H), 1.30 (d, J = 6.7 Hz, 3 H), 0.91 (t, J = 7.4, 3 H).
   ¹³C-NMR: 209.32, 61.13, 50.00, 43.33, 34.79, 34.11, 25.74, 22.29, 22.13, 13.84.
(g) *Synthesis of (±)-tert-butyl (2-((4-oxooctan-2-yl)thio)ethyl)carbamate* **(Compound 7)**
   As described for Compound 6 with tert-butyl (2-mercaptoethyl)carbamate (4.62 g, 25.0 mmol) in THF (20 mL) and (E)-2-octen-4-one (3.28 g, 25.4 mmol) and DBU (0.40 mL, 2.6 mmol) in THF (30 mL). Bulb-to-bulb distillation (170°C, 0.05 mbar) gave 7.01 g (87%) of a colorless oil.
   ¹H-NMR: 4.99 (br. s, 1 H), 3.37-3.23 (m, 3 H), 2.74-2.60 (m, 3 H), 2.54 (dd, J = 17.0, 7.4 Hz, 1 H), 2.47-2.35 (m, 2 H), 1.61-1.52 (m, 2 H), 1.45 (s, 9 H), 1.36-1.27 (m, 2 H), 1.29 (d, J = 6.7 Hz, 3 H), 0.91 (t, J = 7.4 Hz, 3 H).
   ¹³C-NMR: 208.90, 155.80, 79.36, 49.99, 43.35, 40.02, 34.84, 31.10, 28.40, 25.73, 22.30, 21.89, 13.85.
(h) *Synthesis of (*±*)-2-(dodecylsulfinyl)octan-4-one* **(Compound 8)**
   On an ice bath, a solution of (±)-2-(dodecylthio)octan-4-one (1.68 g, 5.1 mmol) in methanol (20 mL) was added to a solution of NaIO4 (1.14 g, 5.3 mmol) in water (11 mL). The reaction mixture was warmed to room temperature, and ethanol (30 mL) was added. The suspension was stirred for 18 h, then extracted with ethyl acetate (100 mL), washed with a saturated aqueous solution of NaCl (50 mL), an aqueous solution of NaHSO₃ (10%, 50 mL), water (50 mL), a saturated aqueous solution of NaHCO₃ (50 mL) and a saturated aqueous solution of NaCl (50 mL). The aqueous phases were each re-extracted with ethyl acetate (70 mL). The combined organic phases were dried (Na₂SO₄), filtered and concentrated and dried under high vacuum for 2 h. Flash chromatography (SiO₂, n-heptane/ethyl acetate 7:3, then 1:1, then 1:4) afforded 0.66 g (37%) of a white solid.
   ¹H-NMR: 3.20 (hex., J = 6.8 Hz, 1 H), 3.04 (dd, J = 18.0, 6.2 Hz, 1 H), 2.71-2.62 (m, 1 H), 2.60 (dd, J = 18.1, 7.4 Hz, 1 H), 2.56-2.39 (m, 3 H), 1.84-1.65 (m, 2 H), 1.62-1.54 (m, 2 H), 1.53-1.37 (m, 2 H), 1.37-1.19 (m, 18 H), 1.23 (d, J = 7.1 Hz, 3 H), 0.91 (t, J = 7.4 Hz, 3 H), 0.88 (t, J = 7.1 Hz, 3 H).
   ¹³C-NMR: 207.96, 48.92, 48.44, 43.61, 43.11, 31.92, 29.62 (2x), 29.54, 29.37, 29.34, 29.21, 28.95, 25.83, 23.16, 22.69, 22.28, 14.13, 13.82, 10.45.
(i) *Synthesis of (±)-2-(dodecylsulfonyl)octan-4-one* **(Compound 9)**
   On an ice bath, a solution of oxone (2 KHSO₅/KHSO₄/K₂SO₄, 4.71 g, 31.0 mmol) in water (25 mL) was added to a solution of 2-(dodecylthio)octan-4-one (2.09 g, 6.4 mmol) in methanol (50 mL). The temperature was allowed to attain 10°C. The ice bath was removed and the suspension was stirred for 2 h. The reaction mixture was extracted with ethyl acetate (100 mL), washed with a saturated aqueous solution of NaCl (50 mL), water (50 m), a saturated aqueous solution of NaHCO₃ (50 mL) and a saturated aqueous solution of NaCl (50 mL). The aqueous phases were each re-extracted with ethyl acetate (50 mL). The combined organic phases were dried (Na₂SO₄), filtered, concentrated and dried under high vacuum for 2 h. Flash chromatography (SiO₂, n-heptane/ethyl acetate 9:1, then 1:1) afforded 1.42 g (62%) of a white solid.
   ¹H-NMR: 3.66-3.57 (m, 1 H), 3.18 (dd, J = 18.1, 3.9 Hz, 1 H), 2.93 (t, J = 8.1 Hz, 2 H), 2.60 (dd, J = 18.3, 8.7 Hz, 1 H), 2.56-2.40 (m, 2 H), 1.93-1.76 (m, 2 H), 1.65-1.54 (m, 2 H), 1.48-1.39 (m, 2 H), 1.39-1.20 (m, 18 H), 1.36 (d, J = 6.7 Hz, 3 H), 0.91 (t, J = 7.4 Hz, 3 H), 0.88 (t, J = 7.4 Hz, 3 H).
   ¹³C-NMR: 206.86, 52.42, 50.41, 43.10, 41.00, 31.91, 29.60, 29.59, 29.51, 29.33, 29.27, 29.07, 28.59, 25.81, 22.69, 22.26, 21.52, 14.49, 14.12, 13.80.
(j) *Synthesis of (±)-2-(dodecylamino)octan-4-one* **(Compound 10)**
   (E)-2-Octen-4-one (1.00 g, 7.9 mmol), 1-dodecylamine (0.98 g, 5.3 mmol) and dimethylsulfoxide (DMSO, 1 mL) were heated at 50°C for 5 h to give the crude compound. A sample (1 mL) was taken, washed with water (3 mL) and extracted with ethyl acetate (4 mL). The remaining volatile compounds were removed by bulb-to-bulb distillation (35°C, 0.2 mbar) for 2 h to give 0.5 g of a yellow oil, still containing small amounts of (E)-2-octen-4-one. Directly removing the volatiles from the remaining crude compound by bulb-to-bulb distillation afforded another 0.89 g of a yellow oil.
   ¹H-NMR: 3.16-3.07 (m, 1 H), 2.65-2.46 (m, 3 H), 2.46-2.35 (m, 3 H), 1.63-1.51 (m, 2 H), 1.50-1.39 (m, 2 H), 1.37-1.20 (m, 20 H), 1.06 (d, J = 6.4 Hz, 3 H), 0.90 (t, J = 7.4 Hz, 3 H), 0.88 (t, J = 6.9 Hz, 3 H).
   ¹³C-NMR: 210.88, 49.91, 49.43, 47.32, 43.39, 31.95, 30.38, 29.70, 29.66, 29.65, 29.62, 29.60, 29.38, 27.45, 25.83, 22.71, 22.34, 20.54, 14.13, 13.87.
(k) *Synthesis of (*±*)-methyl N,S-bis(4-oxooctan-2-yl)-L-cysteinate* **(Compound 11)**
   (R)-Methyl 2-amino-3-mercaptopropanoate hydrochloride (methyl L-cysteinate hydrochloride, 2.23 g, 13.0 mmol), N-ethyl-N-isopropylpropan-2-amine (1.85 g, 14.3 mmol) and (E)-2-octen-4-one (3.30 g, 26.0 mmol) were dissolved in DMSO (35 mL) and stirred at room temperature for 8 d. The reaction mixture was washed with water (150 mL) and extracted with ethyl acetate (150 mL, 2x). The organic phase was washed with water (50 mL, 3x), dried (Na₂SO₄) and concentrated. Drying under vacuum (0.5 mbar for 2 h and at 50°C for 15 min) gave an orange oil. Flash chromatography (SiO₂, n-heptane/ethyl acetate 9:1 to 4:1) and drying under high vacuum (0.07 mbar at 30°C for 2 h) afforded 1.63 g (32%) of a yellow-orange oil (mixture of diastereoisomers).
   ¹H-NMR: 3.75 (t, J = 1.8 Hz, 3 H), 3.54 and 3.49 (q, J = 6.6 and 6.4 Hz, 1 H), 3.34-3.23 (m, 1 H), 3.18-3.09 (m, 1 H), 2.84-2.75 (m, 2 H), 2.75-2.66 (m, 1 H), 2.60 and 2.57 (dd, J = 7.7 and 6.1 Hz, 1 H), 2.55-2.46 (m, 2 H), 2.46-2.34 (m, 4 H), 1.93 (br. s, 1 H), 1.60-1.50 (m, 4 H), 1.36-1.24 (m, 7 H), 1.06 (m, 3 H), 0.94-0.87 (m, 6 H).
   ¹³C-NMR: 210.32, 210.29, 210.04, 208.72, 174.54, 174.52, 174.03, 174.02, 59.52, 59.40, 58.83, 58.73, 52.13, 52.00, 50.33, 49.96, 49.93, 49.92, 48.58, 48.57, 47.61, 43.50, 43.48, 43.34, 43.31, 43.20, 35.77, 35.74, 35.57, 35.54, 34.31, 34.27, 34.24, 25.75, 22.32, 22.30, 21.66, 21.64, 21.21, 21.20, 20.21, 20.19, 13.88, 13.86.
(l) *Synthesis of (±)-2-(benzyloxy)octan-4-one* **(Compound 12**)
   To a solution of (E)-2-octen-4-one (27.00 g, 214.0 mmol) in benzyl alcohol (69.40 g, 642.0 mmol) was added 1,1,3,3-tetramethylguanidine (4.95 g, 43.0 mmol). The reaction mixture was stirred at 40°C for 40 h. Fractional distillation under vacuum (0.08 mbar, 80-109°C) afforded 33.26 g (66%) of a slightly yellow oil.
   ¹H-NMR: 7.37-7.23 (m, 5 H), 4.55 (d, J = 11.5 Hz, 1 H), 4.44 (d, J = 11.5 Hz, 1 H), 4.09-4.00 (m, 1 H), 2.77 (dd, J = 15.7, 7.4 Hz, 1 H), 2.48-2.35 (m, 3 H), 1.58-1.50 (m, 2 H), 1.35-1.24 (m, 2 H), 1.23 (d, J = 6.1 Hz, 3 H), 0.89 (t, J = 7.4 Hz, 3 H).
   ¹³C-NMR: 209.72, 138.56, 128.33, 127.70, 127.54, 71.77, 70.91, 49.89, 43.73, 25.65, 22.28, 19.92, 13.86.
(m) *Synthesis of (±)-4-oxooctan-2-yl undec-10-enoate* **(Compound 13)**
   (i) 4,5-Dichloro-3,6-dioxocyclohexa-1,4-diene-1,2-dicarbonitrile (DDQ, 11.60 g, 51.2 mmol) and water (20 mL) were added to a solution of (±)-2-(benzyloxy)octan-4-one (Compound 12, 10.00 g, 42.7 mmol, prepared as described before) in dichloromethane (180 mL). After 6 h, the mixture was filtered over Celite^{®}. The filtrate was washed with saturated aqueous solutions of NaHCO₃ (50 mL) and NaCl (2x 50 mL). The organic phase was dried (Na₂SO₄) and filtered. The solvent was evaporated, and the residue purified by column chromatography (SiO₂, n-heptane/ethyl acetate 95:5 to 70:30 to 50:50) to give 3.89 g (63%) of (±)-2-hydroxyoctan-4-one as an orange oil.
   ¹H-NMR (400 MHz): 4.28-4.16 (m, 1 H), 3.23 (d, J = 2.8 Hz, 1 H), 2.60 (dd, J = 17.6, 3.2 Hz, 1 H), 2.51 (dd, J = 17.5, 8.7 Hz, 1 H), 2.42 (t, J = 7.4 Hz, 2 H), 1.62-1.51 (m, 2 H), 1.38-1.24 (m, 2 H), 1.19 (d, J = 6.4 Hz, 3 H), 0.91 (t, J = 7.4 Hz, 3 H).
   ¹³C-NMR (100.6 MHz): 212.46, 63.89, 50.45, 43.31, 25.71, 22.37, 22.28, 13.83.
   *(ii)* 10-Undecenoyl chloride (2.32 g, 11.4 mmol) was added at room temperature to a stirred solution of (±)-2-hydroxyoctan-4-one (1.50 g, 10.4 mmol), 4-dimethylaminopyridine (DMAP, 1.67 g, 13.7 mmol) and triethylamine (1.37 g, 13.5 mmol) in dichloromethane (30 mL). The reaction mixture was stirred at room temperature for 30 h, and then poured onto an aqueous solution of HCl (10%, 30 mL), extracted with diethyl ether (30 mL) and washed with saturated aqueous solutions of NaCl (30 mL), NaHCO₃ (30 mL) and NaCl (2x 30 mL). The organic phase was dried (Na₂SO₄), filtered and concentrated. Repetitive column chromatography (SiO₂, n-heptane/ethyl acetate 90:10 and 95:5) afforded 1.23 g (38%) of (±)-(1-methyl-3-oxo-heptyl) undec-10-enoate as a colorless oil.
   ¹H-NMR: 5.86-5.75 (m, 1 H), 5.29 (m, 1 H),5.03-4.90 (m, 2 H), 2.77 (dd, J = 16.0, 7.4 Hz, 1 H), 2.52 (dd, J = 16.0, 6.1 Hz, 1 H), 2.41 (t, J = 7.4, 2 H), 2.24 (t, J = 7.5 Hz, 2 H), 2.07-2.00 (m, 2 H), 1.63-1.50 (m, 4 H), 1.41-1.23 (m, 12 H), 1.26 (d, J = 6.4 Hz, 3 H), 0.90 (t, J = 7.4, 3 H).
   ¹³C-NMR: 207.87, 173.04, 139.17, 114.15, 66.94, 48.58, 43.09, 34.53, 33.79, 29.29, 29.21, 29.08, 29.06, 28.90, 25.67, 24.96, 22.28, 20.11, 13.85.
(n) *Synthesis of (±)-2-ethylhexyl 2-((4-oxooctan-2-yl)thio)acetate* **(Compound 14)**
   (E)-2-octen-4-one (3.00 g, 23.8 mmol) and 2-ethylhexyl 2-mercaptoacetate (4.86 g, 23.8 mmol) were stirred at room temperature for 24 d. Column chromatography (SiO₂, n-heptane/ethyl acetate 90:10) afforded 0.57 g (7%) of a slightly yellow oil as a mixture of diastereoisomers.
   ¹H-NMR: 4.09-4.00 (m, 2 H), 3.45-3.34 (m, 1 H), 3.32-3.24 (m, 2 H), 2.77 (dd, J = 17.0, 5.8 Hz, 1 H), 2.55 (dd, J = 17.0, 8.0 Hz, 1 H), 2.46-2.35 (m, 2 H), 1.65-1.51 (m, 3 H), 1.42-1.23 (m, 16 H), 0.91 (t, J = 7.4 Hz, 3 H), 0.90 (t, J = 7.4 Hz, 3 H).
   ¹³C-NMR: 208.50, 170.81, 67.83, 49.73, 43.16, 38.74, 36.03, 33.02, 30.32, 30.31, 28.90, 25.76, 23.69, 22.97, 22.31, 21.18, 14.05, 13.85, 10.95, 10.94.
(o) *Synthesis of (±)-ethane-1,2-diyl bis(2-((4-oxooctan-2-yl)thio)acetate)* **(Compound 15**) (E)-2-octen-4-one (3.00 g, 23.8 mmol) and ethane-1,2-diyl bis(2-mercaptoacetate) (2.30 g, 10.9 mmol) were stirred at room temperature for 72 h. Consecutive column chromatography (SiO₂, first toluene/methyl tert-butyl ether (MTBE) 90:10, then starting from pure toluene and moving to toluene/MTBE 90:10) and drying under high vacuum (3 h) afforded 3.06 g (60%) of a slightly yellow oil as a mixture of diastereoisomers, containing ca. 6% of 2-(2-mercaptoacetoxy)ethyl 2-((4-oxooctan-2-yl)thio)acetate.
   ¹H-NMR: 4.36 (s, 4 H), 3.47-3.37 (m, 2 H), 3.36-3.28 (m, 4 H), 2.81-2.73 (m, 2 H), 2.56 (dd, J = 17.0, 7.7 Hz, 2 H), 2.47-2.35 (m, 4 H), 1.60-1.51 (m, 4 H), 1.36-1.27 (m, 4 H), 1.30 (d, J = 7.1 Hz, 6 H), 0.91 (t, J = 7.4 Hz, 6 H).
   ¹³C-NMR: 208.45, 170.37, 62.84, 49.63, 43.17, 35.97, 32.70, 25.73, 22.29, 21.23, 13.85.

### Example 2

### Performance of a fabric softener base comprising an invention's compound of formula (I)

The generation of a long-lasting strawberry odor from the present invention's compounds of formula (I) was tested in a fabric softening surfactant emulsion with the following final composition:

| | |
|---|---|
| Stepantex^{®} VL90 A (origin: Stepan) | 12.21 % by weight |
| Calcium chloride (10% aq. solution) | 0.40 % by weight |
| Proxel^{®} GXL (origin: Avecia) | 0.04 % by weight |
| Water | 87.35 % by weight |

### a) Measurement of headspace

In a flask, a solution (0.1 mL) of one of the invention's compounds of formula (I) described in Example 1 (0.055 mmol) in ethanol (10 mL) was added to the fabric softener (0.07 g) in water (0.90 mL). After homogenization, the sample was diluted with demineralized cold tap water (23.3 mL). One cotton sheet (EMPA cotton test cloth Nr. 221, origin: Eidgenössische Materialprufanstalt), pre-washed with an unperfumed detergent powder and cut to ca. 15 × 15 cm sheets, ca. 5.2 g) was added and agitated manually for 3 min, left standing for 2 min, then wrung out by hand, and weighed (ca. 10.0 g) to obtain a constant quantity of residual water. A reference sample (0.1 mL) consisting of an equimolar amount of unmodified (E)-2-octen-4-one (0.055 mmol) in ethanol (10 mL) was added to the fabric softener (0.07 g) in water (0.90 mL) and analyzed the same way. The cotton sheets were line-dried for 1 or 3 days before being analyzed. For the measurements, the sheets were put into a headspace sampling cell (ca. 160 mL inner volume), which were thermostatted at 25°C and exposed to a constant air flow of ca. 200 mL/min. The air was filtered through active charcoal and aspirated through a saturated solution of NaCl (to ensure a constant humidity of the air of ca. 75%). The system was equilibrated during 15 min while adsorbing the volatiles on a waste Tenax^{®} cartridge (filled with 100 mg of Tenax^{®} TA adsorbent resin). Then, seven times consecutively, the volatiles were adsorbed for 15 min on a clean Tenax^{®} cartridge and for 45 min on a waste Tenax^{®} cartridge. Alternatively, one data point was recorded after equilibrating the system for 135 min while adsorbing the volatiles on a waste Tenax^{®} cartridge. Then the volatiles were adsorbed for 15 min on a clean Tenax^{®} cartridge. The waste cartridges were discarded; the other cartridges were desorbed on a Perkin Elmer TurboMatrix ATD desorber coupled to an Agilent Technologies 7890A gas chromatograph equipped with a HP-1 capillary column (30 m, i.d. 0.32 µm, film 0.25 µm) and a FID detector. The volatiles were analyzed using a temperature gradient moving from 60°C to 220°C at 15°C/min. Headspace concentrations (in ng/L air) were obtained by external standard calibrations using different concentrations of the fragrance to be released in ethanol. Each calibration solution (0.2 mL) was injected onto a clean Tenax^{®} cartridge, which was desorbed and analyzed under the same conditions. The results obtained for the evaporation of (E)-2-octen-4-one after a total sampling time of 150 min above dry cotton after line-drying for 1 and 3 days are summarized in Table 1. All data are average values of at least two measurements.

**Table 1: Average headspace concentrations of (E)-2-octen-4-one measured after sampling for 150 min on dry cotton after line-drying for 1 and 3 days.**

| Compound | Concentration of (E)-2-octen-4-one [ng/L] measured after line-drying for 1 day | Concentration of (E)-2-octen-4-one [ng/L] measured after line-drying for 3 days |
|---|---|---|
| Reference | 1.6 | 0.8 |
| Compound 1 | 35.3 | 24.7 |
| Compound 2 | 54.1 | 33.0 |
| Compound 3 | 5.1 | 16.1 |
| Compound 4 | 4.1 | 10.9 |
| Compound 5 | 5.7 | 10.8 |
| Compound 6 | 6.8 | 6.7 |
| Compound 7 | 13.1 | 13.3 |
| Compound 8 | 77.7 | 60.5 |
| Compound 9 | 44.9 | 18.6 |
| Compound 10 | 8.4 | 6.6 |
| Compound 11 | 52.2 | 14.1 |
| Compound 13 | 10.6 | 8.2 |
| Compound 14 | 20.8 | 19.1 |
| Compound 15 | 7.3 | 9.3 |

The compounds according to formula (I) released at least equivalent, but mostly higher amounts of (E)-2-octen-4-one into the headspace above dry cotton than the reference sample. Compounds of formula (I) according to the present invention are thus capable of imparting a long-lasting strawberry odor to a cotton surface.

### b) Sensory evaluation

Cotton terry towels (30 pieces, 18 cm × 18 cm, about 30 g each) were washed with 30 g of unperfumed detergent in a European washing machine (Miele Novotronic W300-33CH) at 40°C using the short cycle program. The wash was followed by a rinse at 900 rpm with 12.7 g of above concentrated fabric-softener comprising Compound 2 or an equimolar amount of (E)-2-octen-4-one. The terry towels were then line dried for 24 h before being evaluated by a panel of 20 trained panelists. The panelists were asked to rate the odor intensity of the towels on a scale from 1 to 7, 1 corresponding to odorless and 7 corresponding to a very strong odor. The results are shown in Table 2 below.

**Table 2: Olfactive performance in a fabric softener application**

| Overall Perfume Intensity | Line Drying | |
|---|---|---|
| | 1day | 3 days |
| Softener comprising Compound 2 | 4.1 | 4.1 |
| Softener comprising an equimolar amount of (E)-2-octen-4-one | 3.1 | 2. 7 |

The performance on dry fabric from the invention's compound releasing (E)-2-octen-4-one was higher than (E)-2-octen-4-one, in particular after 3 days. Compounds of formula (I) according to the present invention are thus capable of imparting a long-lasting strawberry odor confirming the above-mentioned-headspace analyses reported in Table 1.

### Example 3

### Performance of an all-purpose hard surface cleaner formulation comprising an invention's compound of formula (I)

The generation of a long-lasting strawberry odor from the present invention's compounds of formula (I) was tested in an all-purpose surface cleaner (APC). An APC formulation with the following final composition has been prepared:

| | |
|---|---|
| Neodol^{®} 91-8 (origin: Shell Chemicals) | 5.0 % by weight |
| Marlon^{®} A 375 (origin: Hüls AG) | 4.0 % by weight |
| Sodium cumolsulphonate | 2.0 % by weight |
| Kathon^{®} CG (origin: Rohm and Haas) | 0.2 % by weight |
| Water | 88.8 % by weight |

In a flask, one of the invention's compounds of formula (I) (0.0369 mmol) was dissolved in ethanol (100 µm). Then the APC formulation (3.0 g) was added and the sample shaken gently. An aliquot of these samples (1 mL) was pipetted off and diluted with demineralized tap water (9 mL). A film of this solution (0.75 mL) was pipetted onto a porous ceramic plate (ca. 5 × 10 cm), covered with a crystallizing dish (2.5 L) and left standing at room temperature. Similarly, a reference sample consisting of unmodified (E)-2-octen-4-one (0.0369 mmol) instead of one of the invention's compounds of formula (I) in ethanol (100 µm) was prepared and processed the same way.

After one day, each of the ceramic plates was placed inside a headspace sampling cell (ca. 625 mL) and exposed to a constant air flow of ca. 200 mL/min. The air was filtered through active charcoal and aspirated through a saturated aqueous solution of NaCl (to ensure a constant humidity of the air of ca. 75%). The headspace system was equilibrated for 15 min by adsorbing the volatiles onto a waste Tenax^{®} cartridge. Then the volatiles were alternately adsorbed for 10 min onto a clean Tenax^{®} cartridge and for 20 min onto a waste Tenax^{®} cartridge (6x). The waste cartridges were discarded; the clean cartridges were desorbed and analyzed as described in Example 2. All measurements were performed at least twice. The average headspace concentrations of (E)-2-octen-4-one released from the compounds of formula (I) as prepared in Example 1 or from the reference sample after 115 min of sampling above the porous ceramic plates are listed in Table 3. Table 3 also indicates the factors of increase of (E)-2-octen-4-one released from the invention's compounds of formula (I) with respect to the reference sample.

**Table 3: Average headspace concentrations of (E)-2-octen-4-one measured after 115 min of sampling in an APC application above ceramic plates and factors of increase of the headspace concentrations released from the invention's compounds of formula (I) with respect to the reference sample.**

| Compound | Concentration of (E)-2-octen-4-one [ng/L] released from the invention's compounds of formula (I) | Factor of increase with respect to unmodified (E)-2-octen-4-one as the reference |
|---|---|---|
| Reference | 1.4 | --- |
| Compound 2 | 17.2 | ca. 12 |
| Compound 5 | 60.4 | ca. 43 |
| Compound 7 | 32.5 | ca. 23 |
| Compound 12 | 8.7 | ca. 6 |
| Compound 13 | 52.7 | ca. 40 |
| Compound 14 | 12.4 | ca. 9 |
| Compound 15 | 40.3 | ca. 29 |

After 1 day, the invention's compounds of formula (I) as prepared in Example 1 release more (E)-2-octen-4-one into the headspace than the reference sample. The invention's compounds of formula (I) are thus able to provide a long-lasting and substantive strawberry odor to a hard surface from an APC application.

### Example 4

### Performance of a hair conditioning formulation comprising an invention's compound of formula (I)

The generation of a long-lasting strawberry odor from the present invention's compounds of formula (I) was tested in a hair conditioning application. A hair conditioning formulation with the following final composition has been prepared:

| | |
|---|---|
| Dehyquart^{®} C 4046 (origin: BASF) | 5.00 % by weight |
| Glycerin (85%) (origin: Brenntag) | 2.00 % by weight |
| Paraffinum Perliquidum (origin: Acros) | 2.00 % by weight |
| Genamin^{®} CTAC (origin: Clariant) | 1.00 % by weight |
| Xiameter MEM-949 Cationic Emulsion (origin: Xiameter) | 1.00 % by weight |
| Jaguar^{®} C14 S (origin: Lubrizol) | 0.30 % by weight |
| Kathon^{®} CG (origin: Rohm and Haas) | 0.08 % by weight |
| EDTA B Powder (origin: BASF) | 0.05 % by weight |
| Water (deionized) | 88.57 % by weight |

A solution of one of the present invention's compounds of formula (I) in ethanol was prepared by weighing precisely 0.4 mmol of the compound into a volumetric flask (5 mL) and filling up with ethanol. Similarly, a reference solution containing an equimolar amount of (E)-2-octen-4-one was prepared.

The hair conditioner formulation described above (920 mg) was weighed in a sample tube (3 mL), then the ethanol solution (100 µL) containing one of the invention's compounds of formula (I) or (E)-2-octen-4-one (reference) were added. The tube was closed, shaken (50x) and centrifuged with a manual centrifuge (at ca. 3500 rpm) during 30 s.

A Caucasian hair swatch (origin: Kerling International Haarfabrik GmbH, ca. 10 cm long, about 0.5 g) was rinsed and rubbed under tap water at 37°C with a flow of ca. 2 L/min for 30 s, and the excess of water was squeezed out with the fingertips. An unperfumed shampoo (0.1 g) was spread onto the hair swatch, which was washed for 30 s. Then the shampoo was rinsed off with tap water at 37°C for 30 s, and the excess of water was squeezed out with the fingertips. Then the hair conditioning formulation (0.1 g), containing either one of the invention's compounds according to formula (I) or (E)-2-octen-4-one as the reference, was spread onto the hair swatch. The hair swatch was gently rubbed between the fingertips for 1 min, combed once and line-dried.

After 6 h, the hair swatch was combed (l0x) and fixed with adhesive tape inside a thermostatted (25°C) headspace sampling cell with an inner volume of ca.165 mL. A constant flow of air (200 mL/min) was pumped across the sample. The incoming air was filtered through active charcoal, and through a saturated aqueous solution of NaCl. The system was equilibrated for 10 min by absorbing the volatiles onto a waste Tenax^{®} cartridge. Then the volatiles were adsorbed for 10 min onto a first clean Tenax^{®} cartridge and for another 10 min onto a second clean Tenax^{®} cartridge. Then the pump was stopped. The hair swatch was left inside the headspace sampling cell without connecting a cartridge. After 24 h a waste Tenax^{®} cartridge was connected, the pump was switched on and the system was equilibrated for 10 min. Then the volatiles were consecutively adsorbed for 10 min onto two clean Tenax^{®} cartridges. The waste cartridges were discarded; the clean cartridges were desorbed and analyzed as described in Example 2. All measurements were performed at least twice.

The average headspace concentrations of (E)-2-octen-4-one released from the compounds of formula (I) as prepared in Example 1 or from the reference sample (desorbed from the first cartridge) after 6 h and after 24 h are listed in Table 4. Table 4 also indicates the factors of increase of (E)-2-octen-4-one released from the invention's compounds of formula (I) with respect to the reference sample.

**Table 4: Average headspace concentrations of (E)-2-octen-4-one measured in a hair conditioning application on a hair swatch after 6 h and 24 h and factors of increase of the headspace concentrations released from the invention's compounds of formula (I) with respect to the reference sample.**

| Compound | Concentration of (E)-2-octen-4-one [ng/L] released from the invention's compounds of formula (I) after 6 h | Factor of increase with respect to unmodified (E)-2-octen-4-one as the reference after 6 h |
|---|---|---|
| Reference | 1.6 | --- |
| Compound 2 | 4.1 | ca. 2.5 |
| Compound 5 | 2.2 | ca. 1.4 |
| Compound 7 | 3.0 | ca. 1.9 |
| Compound 13 | 7.6 | ca. 4.8 |
| | after 24 h | after 24 h |
| Reference | 3.8 | --- |
| Compound 2 | 9.1 | ca. 2.4 |
| Compound 5 | 4.4 | ca. 1.2 |
| Compound 7 | 5.4 | ca. 1.4 |
| Compound 13 | 25.3 | ca. 6.7 |

Both after 6 h and after 24 h the invention's compounds of formula (I) as prepared in Example 1 release more (E)-2-octen-4-one into the headspace than the reference sample. The invention's compounds of formula (I) are thus able to provide a long-lasting and substantive strawberry odor to hair from a hair conditioning application.

### Example 5

### Preparation of a liquid detergent comprising the invention's compound

**Table 5: Composition of the liquid detergent formulation**

| Ingredients | Concentration [wt%] |
|---|---|
| Sodium C14-17 Alkyl Sec Sulfonate¹⁾ | 7 |
| Fatty acids, C12-18 and C18-unsaturated²⁾ | 7.5 |
| C12/14 fatty alcohol polyglycol ether with 7 mol EO³⁾ | 17 |
| Triethanolamine | 7.5 |
| Propylene Glycol | 11 |
| Citric acid | 6.5 |
| Potassium Hydroxyde | 9.5 |
| Properase L⁴⁾ | 0.2 |
| Puradax EG L⁴⁾ | 0.2 |
| Purastar ST L⁴⁾ | 0.2 |
| Acrylates/Steareth-20 Methacrylate structuring Crosspolymer⁵⁾ | 6 |
| Deionized Water | 27.4 |

| | |
|---|---|
| 1) Hostapur SAS 60; Origin: Clariant 2) Edenor K 12-18; Origin: Cognis 3) Genapol LA 070; Origin: Clariant 4) Origin: Genencor International 5) Aculyn 88; Origin: Dow Chemical | |

The liquid detergent is prepared by adding 0.01 to 5% preferably 0.5 to 1.5% by weight, relative to the total weight of the liquid detergent, of the invention's Compound 2 into the unperfumed liquid detergent formulation of Table 5 under gentle shaking.

### Example 6

### Preparation of a transparent isotropic shampoo comprising the invention's composition

**Table 6: Composition of the transparent isotropic shampoo formulation**

| **Phases** | **Ingredients** | **Concentration [wt%]** |
|---|---|---|
| **A** | Water deionized | 44.4 |
| | Polyquaternium-10 ¹⁾ | 0.3 |
| | Glycerin 85% ²⁾ | 1 |
| | DMDM Hydantoin ³⁾ | 0.2 |
| **B** | Sodium Laureth Sulfate ⁴⁾ | 28 |
| | Cocamidopropyl Betaine ⁵⁾ | 3.2 |
| | Disodium Cocoamphodiacetate ⁶⁾ | 4 |
| | Ethoxy (20) Stearyl Alcohol ⁶⁾ | 1 |
| **C** | Sodium Laureth Sulfate ⁴⁾ | 3 |
| | Glyceryl Laureate ⁷⁾ | 0.2 |
| **D** | Water deionized | 1 |
| | Sodium Methylparaben ⁸⁾ | 0.1 |
| **E** | Sodium Chloride 10% aqueous sol. | 15 |
| | Citric acid 10% aqueous sol. till pH 5.5-6 | q.s. |

| | | |
|---|---|---|
| 1) Ucare Polymer JR-400, Origin: Noveon 2) Origin: Schweizerhall 3) Glydant, Origin: Lonza 4) Texapon NSO IS, Origin: Cognis 5) Tego Betain F 50, Origin: Evonik 6) Amphotensid GB 2009, Origin: Zschimmer & Schwarz 7) Monomuls 90 L-12, Origin: Gruenau 8) Nipagin Monosodium, Origin: NIPA | | |

The shampoo is prepared by dispersing Polyquaternium-10 in water. The remaining ingredients of phase A are mixed separately by addition of one after the other while mixing well after each adjunction. This pre-mix is added to the Polyquaternium-10 dispersion and mixed for another 5 min. Then, the premixed phase B and the premixed Phase C are added (Monomuls 90L-12 is heated to melt in Texapon NSO IS) while agitating. Phase D and Phase E are added while agitating. PH is adjusted with citric acid solution till pH: 5.5 - 6.0 leading to an unperfumed shampoo formula.

The perfumed shampoo is prepared by adding 0.01 to 5% preferably 0.5 to 1.5% by weight of the invention's Compound 2, relative to the total weight of the shampoo, into the unperfumed shampoo formulation of Table 6 under gentle shaking.

### Example 7

### Preparation of a structured shower gel comprising the invention's composition

**Table 7: Composition of the shower gel formulation**

| **Ingredients** | **Amount (% wt)** |
|---|---|
| WATER deionised | 49.350 |
| Tetrasodium EDTA ¹⁾ | 0.050 |
| Acrylates Copolymer²⁾ | 6.000 |
| Sodium C12-C15 Pareth Sulfate³⁾ | 35.000 |
| Sodium Hydroxide 20% aqueous solution | 1.000 |
| Cocamidopropyl Betaine⁴⁾ | 8.000 |
| Methylchloroisothiazolinone and Methylisothiazolinone⁵⁾ | 0.100 |
| Citric Acid (40%) | 0.500 |

| | |
|---|---|
| 1) EDETA B POWDER; trademark and origin: BASF 2) CARBOPOL AQUA SF-1 POLYMER; trademark and origin: NOVEON 3) ZETESOL AO 328 U; trademark and origin: ZSCHIMMER & SCHWARZ 4) TEGO-BETAIN F 50; trademark and origin: GOLDSCHMIDT 5) KATHON CG; trademark and origin: ROHM & HASS | |

The shower gel is prepared by adding 0.01 to 5% preferably 0.5 to 1.5% by weight of the invention's Compound 2, relative to the total weight of the shower gel, into the unperfumed shower gel formulation of Table 7 under gentle shaking.

### Example 8

### Preparation of a transparent shower gel comprising the invention's composition

**Table 8: Composition of the transparent shower gel formulation**

| **Ingredients** | **Concentration (% wt)** |
|---|---|
| WATER deionized | 52.40 |
| Tetrasodium EDTA ¹⁾ | 0.10 |
| Sodium Benzoate | 0.50 |
| Propylene Glycol | 2.00 |
| Sodium C12-C15 Pareth Sulfate²⁾ | 35.00 |
| Cocamidopropyl Betaine³⁾ | 8.00 |
| Polyquaternium-7⁴⁾ | 0.20 |
| Citric Acid (40%) | 1.00 |
| Sodium Chloride | 0.80 |

| | |
|---|---|
| 1) EDETA B POWDER; trademark and origin: BASF 2) ZETESOL AO 328 U; trademark and origin: ZSCHIMMER & SCHWARZ 3) TEGO-BETAIN F 50; trademark and origin: GOLDSCHMIDT 4) MERQUAT 550; trademark and origin: LUBRIZOL | |

The transparent shower gel is prepared by adding 0.01 to 5% preferably 0.5 to 1.5% by weight of the invention's Compound 2, relative to the total weight of the shower gel, into the unperfumed shower gel formulation of Table 8 under gentle shaking.

### Example 9

### Preparation of milky shower gel comprising the invention's composition

**Table 9: Composition of the milky shower gel formulation**

| **Ingredients** | **Concentration (% wt)** |
|---|---|
| WATER deionized | 50.950 |
| Tetrasodium EDTA ¹⁾ | 0.050 |
| Sodium Benzoate | 0.500 |
| Glycerin 86% | 3.500 |
| Sodium Laureth Sulfate ²⁾ | 27.000 |
| Polyquaternium-7³⁾ | 1.000 |
| Coco-Betaine⁴⁾ | 6.000 |
| PEG-120 Methyl Glucose trioleate⁵⁾ | 1.000 |
| Citric Acid (40%) | 1.000 |
| Glycol Distearate & Laureth-4 & Cocamidopropyl Betaine⁶⁾ | 3.000 |
| Sodium Chloride 20% | 5.000 |
| PEG-40 Hydrogenated Castor Oil⁷⁾ | 1.000 |

| | |
|---|---|
| 1) EDETA B POWDER; trademark and origin: BASF 2) Texapon NSO IS; trademark and origin: COGNIS 3) MERQUAT 550; trademark and origin: LUBRIZOL 4) DEHYTON AB-30; trademark and origin: COGNIS 5) GLUCAMATE LT; trademark and origin: LUBRIZOL 6) EUPERLAN PK 3000 AM; trademark and origin: COGNIS 7) CREMOPHOR RH 40; trademark and origin: BASF | |

The transparent shower gel is prepared by adding 0.01 to 5% preferably 0.5 to 1.5% by weight of the invention's Compound 9, relative to the total weight of the shower gel, into the unperfumed shower gel formulation of Table 8 under gentle shaking.

### Example 10

### Preparation of a hand dishwash comprising the invention's composition

**Table 10: Composition of the hand dishwash formulation**

| **Ingredients** | **Amount (% wt)** |
|---|---|
| Linear alkylbenzene sulfonic acid ⁽¹⁾ | 20 |
| Diethanolamide ⁽²⁾ | 3.5 |
| Sodium Hydroxide (50%) ⁽³⁾ | 3.4 |
| Secondary alcohol ethoxolate ⁽⁴⁾ | 2.5 |
| Sodium xylene sulfonate | 6.3 |
| Water | 64.3 |

| | |
|---|---|
| 1) Biosoft S-118^{®}; trademark and origin : Stepan Company 2) Ninol 40-CO^{®}; trademark and origin : Stepan Company 3) Stepanate SXS^{®}; trademark and origin : Stepan Company 4) Tergitol 15-S-9^{®}; trademark and origin : Dow Chemical Company | |

Water with sodium hydroxide and diethanolamide are mixed. LAS is added. After the LAS is neutralized, the remaining ingredients are added. The pH was checked (=7-8) and adjusted if necessary.

The perfumed hand dishwash is prepared by adding 0.01 to 5% preferably 0.5 to 1.5% by weight of the invention's Compound 2, relative to the total weight of the hand dishwash, into the unperfumed hand dishawash formulation of Table 10 under gentle shaking.

## Claims

1. A compound of formula 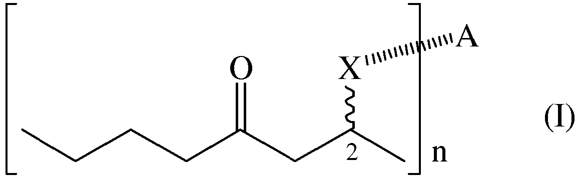 wherein
n represents an integer varying from 1 to 6;
A represents an n-valent C₂ to C₂₂ hydrocarbon group, optionally comprising one to eight ether groups and/or one or two functional groups selected from the group consisting of alcohol, ketone, aldehyde, ester, thioether, carboxylic acid, alkali carboxylate, amine, amide, carbamate, nitrile or thiol; and
X represents a group of formula (i) to (vi) 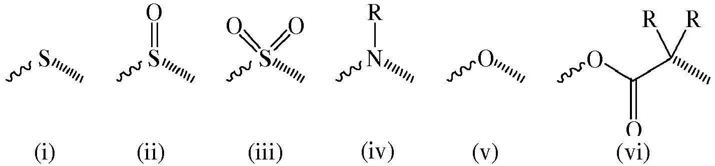
in which formulae the wavy line indicates the location of the bond between carbon 2 and X and the hatched line indicates the location of the bond between X and A, R representing independently of each other a hydrogen atom or a C₁ to C₄ linear or branched hydrocarbon group, provided that the hatched line is not directly linked to a heteroatom or a carbonyl functional group of A, and provided that 2-(propylthio)octan-4-one, 4-oxooctan-2-yl 4-(benzyloxy)benzoate, 2-(methoxymethoxy)octan-4-one, 2-((2-methoxyethoxy)methoxy)octan-4-one, 2-(ethylamino)octan-4-one and 2-(benzylamino)octan-4-one are excluded.

2. The compound according to claim 1, **characterized in that** X represents a group of
formula (i) to (iv) or (vi), preferably a group of formula (i).

3. The compound according to claims 1 or 2, **characterized in that** A represents an n-valent C₂ to C₁₈ hydrocarbon group, optionally comprising one to four ether groups and/or one or two functional groups selected from the group consisting of alcohol, ketone, aldehyde, ester, carboxylic acid, amine or carbamate.

4. The compound according to any one of claims 1 to 3, **characterized in that** n is an integer varying between 1 and 2, preferably n is 1.

5. The compound according to any one of claims 1 to 4 **characterized in that** said compound is a 2-(alkylthio)octan-4-one, a 2-(alkylsulfinyl)octan-4-one, a 2-(alkylsulfonyl)octan-4-one or a 4-oxooctan-2-yl alkanoate or alkenoate, with the size of the alkyl or alkenyl group varying between C₆ and C₁₈.

6. Use of a compound of formula (I) according to any one of the claims 1 to 5 as perfuming ingredient to provide a long-lasting strawberry odor to the environment.

7. The use according to claim 6 **characterized in that** said compound of formula (I) is used in combination with at least one ingredient selected amongst oct-2-en-4-one, 2,5-dimethyl-4-hydroxy-2*H*-furan-3-one, 5-ethyl-4-hydroxy-2-methyl-3(2*H*)-furanone, 2,5-dimethyl-4-oxo-4,5-dihydrofuran-3-yl acetate, ethyl 3-methyl-3-phenyloxirane-2-carboxylate, ethyl 3-phenyloxirane-2-carboxylate, methyl 2-acetamidobenzoate or 3-phenylpropyl 3-methylbutanoate, 2-methyl-4-oxo-4*H*-pyran-3-yl propionate, 2-methylpent-2-enoic acid or (2S,5S)-2-(*tert*-butyl)-5-methyl-2-propyltetrahydrofuran .

8. A method to confer, enhance, improve or modify the strawberry odor properties of a perfuming composition or of a perfumed article, which method comprises adding to said composition or article an effective amount of at least a compound of formula (I) according to any of the claims 1 to 5.

9. A perfuming composition comprising:
i) as perfuming ingredient, at least one compound of formula (I) as defined in any one of claims 1 to 5;
ii) at least one ingredient selected from the group consisting of a perfumery carrier and a perfumery base; and
iii) optionally at least one perfumery adjuvant.

10. The perfuming composition according to claim 9, **characterized in that** it further comprises at least one compound selected amongst the isothiazolones of formula 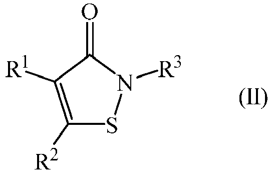
wherein R¹ and R² represent, separately and independently of each other, a hydrogen atom, a halogen atom, preferably chlorine, a C₁-C₄ linear or branched alkyl group, an amino group or a benzylamino group; or, alternatively, R¹ and R² are taken together to represent a phenyl or pyridine ring, possibly substituted with one to four C₁-C₄ linear or branched alkyl or alkenyl groups and/or one to two halogen atoms, preferably chlorine atoms; and
R³ represents a hydrogen atom, an alkali metal atom, in particular Na or K, a phenyl or benzyl group possibly substituted with one or two halogen atoms and/or one or two methyl, trifluoromethyl, methoxy or amino groups, an amine group, or a C₁-C₈ unsaturated, linear, branched or cyclic hydrocarbon group possibly substituted with one or two nitrogen, oxygen or halogen atoms.

11. A perfumed consumer product which comprises:
i) as perfuming ingredient, at least one compound of formula (I), as defined in any one of claims 1 to 5, or a perfuming composition, as defined in claims 9 or 10; and
ii) a perfumery consumer base.

12. The perfumed consumer product according to claim 11, **characterized in that** the perfumery consumer product is a perfume, a fabric care product, a body-care product, an air care product or a home care product.

13. The perfumed consumer product according to claim 11, **characterized in that** the perfumery consumer product is a fine perfume, a liquid or solid detergent, a fabric softener, a fabric refresher, an ironing water, a shampoo, a coloring preparation, a hair spray, a deodorant or antiperspirant, a perfumed soap, shower or bath mousse, oil or gel, a hygiene product, an air freshener, a "ready to use" powdered air freshener or a hard-surface detergent.

14. A method of imparting a long-lasting or substantive strawberry odor to surfaces, such as hard surfaces, fabric, skin or hair, by adding at least one compound of formula (I) according to claims 1 to 5 to perfuming compositions or perfumed articles and applying them to the corresponding targeted surface.

## Patentansprüche

1. Verbindung der Formel 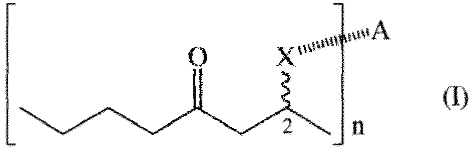 wobei
n eine ganze Zahl, variierend von 1 bis 6, darstellt;
A eine n-wertige C₂- bis C₂₂-Kohlenwasserstoffgruppe darstellt, wahlweise umfassend ein bis acht Ethergruppen und/oder ein oder zwei funktionelle Gruppen, ausgewählt aus der Gruppe, bestehend aus Alkohol, Keton, Aldehyd, Ester, Thioether, Carbonsäure, Alkalicarboxylat, Amin, Amid, Carbamat, Nitril oder Thiol; und
X eine Gruppe der Formel (i) bis (vi) darstellt 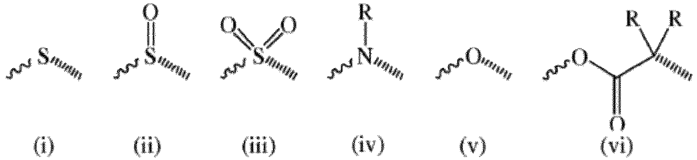
in welchen Formeln die Wellenlinie die Stelle der Bindung zwischen Kohlenstoff 2 und X angibt und die schraffierte Linie die Stelle der Bindung zwischen X und A angibt, wobei R unabhängig voneinander ein Wasserstoffatom oder eine lineare oder verzweigte C₁- bis C₄-Kohlenwasserstoffgruppe darstellen, mit der Maßgabe, dass die schraffierte Linie nicht direkt mit einem Heteroatom oder einer funktionellen Carbonylgruppe von A verknüpft ist, und mit der Maßgabe, dass 2-(Propylthio)octan-4-on, 4-Oxooctan-2-yl-4-(benzyloxy)benzoat, 2-(Methoxymethoxy)octan-4-on, 2-((2-Methoxyethoxy)methoxy)octan-4-on, 2-(Ethylamino)octan-4-on und 2-(Benzylamino)octan-4-on ausgeschlossen sind.

2. Verbindung nach Anspruch 1, **dadurch gekennzeichnet, dass** X eine Gruppe der Formel (i) bis (iv) oder (vi), vorzugsweise eine Gruppe der Formel (i), darstellt.

3. Verbindung nach Anspruch 1 oder 2, **dadurch gekennzeichnet, dass** A eine n-wertige C₂- bis C₁₈-Kohlenwasserstoffgruppe darstellt, wahlweise umfassend ein bis vier Ethergruppen und/oder ein oder zwei funktionelle Gruppen, ausgewählt aus der Gruppe, bestehend aus Alkohol, Keton, Aldehyd, Ester, Carbonsäure, Amin oder Carbamat.

4. Verbindung nach einem der Ansprüche 1 bis 3, **dadurch gekennzeichnet, dass** n eine ganze Zahl, variierend zwischen 1 und 2, ist, vorzugsweise n 1 ist.

5. Verbindung nach einem der Ansprüche 1 bis 4, **dadurch gekennzeichnet, dass** die Verbindung ein 2-(Alkylthio)octan-4-on, ein 2-(Alkylsulfinyl)octan-4-on, ein 2-M&C/SELY/KRGA/13.01.2022 (Alkylsulfonyl)octan-4-on oder ein 4-Oxooctan-2-ylalkanoat oder -alkenoat ist, wobei die Größe der Alkyl- oder Alkenylgruppe zwischen C₆ und C₁₈ variiert.

6. Verwendung einer Verbindung der Formel (I), nach einem der Ansprüche 1 bis 5, als parfümierende Ingredienz, um der Umgebung einen langanhaltenden Erdbeergeruch zu verschaffen.

7. Verwendung nach Anspruch 6, **dadurch gekennzeichnet, dass** die Verbindung der Formel (I) in Kombination mit mindestens einer Ingredienz verwendet wird, die ausgewählt ist unter Oct-2-en-4-on, 2,5-Dimethyl-4-hydroxy-2*H*-furan-3-on, 5-Ethyl-4-hydroxy-2-methyl-3(2*H*)-furanon, 2,5-Dimethyl-4-oxo-4,5-dihydrofuran-3-ylacetat, Ethyl-3-methyl-3-phenyloxiran-2-carboxylat, Ethyl-3-phenyloxiran-2-carboxylat, Methyl-2-acetamidobenzoat oder 3-Phenylpropyl-3-methylbutanoat, 2-Methyl-4-oxo-4*H*-pyran-3-ylpropionat, 2-Methylpent-2-ensäure oder (2S,5S)-2-(*tert*-Butyl)-5-methyl-2-propyltetrahydrofuran.

8. Verfahren zum Verleihen, Verstärken, Verbessern oder Verändern der Erdbeergeruchseigenschaften einer parfümierenden Zusammensetzung oder eines parfümierten Artikels, welches Verfahren Hinzufügen, zu der Zusammensetzung oder dem Artikel, einer wirksamen Menge von mindestens einer Verbindung der Formel (I) nach einem der Ansprüche 1 bis 5 umfasst.

9. Parfümierende Zusammensetzung, umfassend:
i) als parfümierende Ingredienz, mindestens eine Verbindung der Formel (I) wie in einem der Ansprüche 1 bis 5 definiert;
ii) mindestens eine Ingredienz, ausgewählt aus der Gruppe, bestehend aus einem Parfümträger und einer Parfümbasis; und
iii) wahlweise mindestens einen Parfümhilfsstoff.

10. Parfümierende Zusammensetzung nach Anspruch 9, **dadurch gekennzeichnet, dass** diese weiterhin mindestens eine Verbindung umfasst, die ausgewählt ist unter den Isothiazolonen der Formel 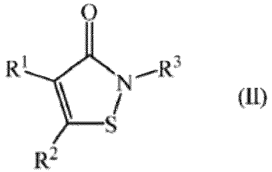 wobei R¹ und R², gesondert und unabhängig voneinander, ein Wasserstoffatom, ein Halogenatom, vorzugsweise Chlor, eine lineare oder verzweigte C₁-C₄-Alkylgruppe, eine Aminogruppe oder eine Benzylaminogruppe darstellen; oder alternativ R¹ und R² zusammengenommen sind unter Darstellung eines Phenyl- oder Pyridinrings, gegebenenfalls substituiert mit ein bis vier linearen oder verzweigten C₁-C₄-Alkyl- oder Alkenylgruppen und/oder ein bis zwei Halogenatomen, vorzugsweise Chloratomen; und M&C/SELY/KRGA/13.01.2022
R³ ein Wasserstoffatom, ein Alkalimetallatom, insbesondere Na oder K, eine Phenyl- oder Benzylgruppe, gegebenenfalls substituiert mit ein oder zwei Halogenatomen und/oder ein oder zwei Methyl-, Trifluormethyl-, Methoxy- oder Aminogruppen, eine Amingruppe oder eine ungesättigte, lineare, verzweigte oder cyclische C₁-C₈-Kohlenwasserstoffgruppe, gegebenenfalls substituiert mit ein oder zwei Stickstoff-, Sauerstoff- oder Halogenatomen, darstellt.

11. Parfümiertes Verbraucherprodukt, das umfasst:
i) als parfümierende Ingredienz, mindestens eine Verbindung der Formel (I), wie in einem der Ansprüche 1 bis 5 definiert, oder eine parfümierende Zusammensetzung, wie in Anspruch 9 oder 10 definiert; und
ii) eine Parfüm-Verbraucherprodukt-Basis.

12. Parfümiertes Verbraucherprodukt nach Anspruch 11, **dadurch gekennzeichnet, dass** das Parfüm-Verbraucherprodukt ein Parfüm, ein Textilpflegeprodukt, ein Körperpflegeprodukt, ein Luftpflegeprodukt oder ein Haushaltspflegeprodukt ist.

13. Parfümiertes Verbraucherprodukt nach Anspruch 11, **dadurch gekennzeichnet, dass** das Parfüm-Verbraucherprodukt besteht in einem Feinparfüm, einem flüssigen oder festen Detergens, einem Weichspüler, einem Textilerfrischer, einem Bügelwasser, einem Shampoo, einem Färbepräparat, einem Haarspray, einem Deodorant oder Antitranspirant, einer parfümierten Seife, Dusch- oder Badeschaum, -öl oder -gel, einem Hygieneprodukt, einem Lufterfrischer, einem "gebrauchsfertigen" pulverförmigen Lufterfrischer oder einem Reinigungsmittel für harte Flächen.

14. Verfahren zur Verleihung eines langanhaltenden oder substantiellen Erdbeergeruchs an Flächen, wie beispielsweise harte Flächen, Textil, Haut oder Haar, durch Hinzufügen mindestens einer Verbindung der Formel (I) nach Ansprüchen 1 bis 5 zu parfümierenden Zusammensetzungen oder parfümierten Artikeln und Anwenden von diesen auf die entsprechende angezielte Fläche.

## Revendications

1. Composé de formule 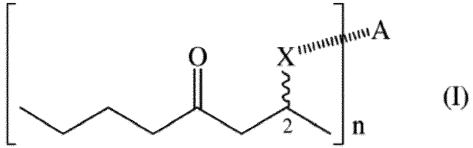
dans lequel
n représente un entier variant de 1 à 6 ;
A représente un groupe hydrocarboné en C₂ à C₂₂ n-valent, comprenant facultativement un à huit groupes éther et/ou un ou deux groupes fonctionnels sélectionnés dans le groupe constitué par : alcool, cétone, aldéhyde, ester, thioéther, acide carboxylique, carboxylate alcalin, amine, amide, carbamate, nitrile ou thiol ; et
X représente un groupe de formule (i) à (vi) 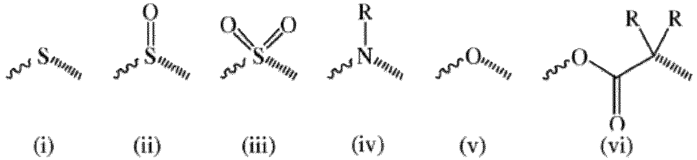
formules dans lesquelles le trait ondulé indique l'emplacement de la liaison entre le carbone 2 et X et le trait hachuré indique l'emplacement de la liaison entre X et A, les R représentant indépendamment l'un de l'autre un atome d'hydrogène ou un groupe hydrocarboné linéaire ou ramifié en C₁ à C₄, sous réserve que le trait hachuré ne soit pas directement lié à un hétéroatome ou un groupe fonctionnel carbonyle de A, et sous réserve que la 2-(propylthio)octan-4-one, le 4-(benzyloxy)benzoate de 4-oxooctan-2-yle, la 2-(méthoxyméthoxy)octan-4-one, la 2-((2-méthoxyéthoxy)méthoxy)octan-4-one, la 2-(éthylamino)octan-4-one et la 2-(benzylamino)octan-4-one soient exclus.

2. Composé selon la revendication 1, **caractérisé en ce que** X représente un groupe de formule (i) à (iv) ou (vi), de préférence un groupe de formule (i).

3. Composé selon les revendications 1 ou 2, **caractérisé en ce que** A représente un groupe hydrocarboné en C₂ à C₁₈ n-valent, comprenant facultativement un à quatre groupes éther et/ou un ou deux groupes fonctionnels sélectionnés dans le groupe constitué par : alcool, cétone, aldéhyde, ester, acide carboxylique, amine ou carbamate.

4. Composé selon l'une quelconque des revendications 1 à 3, **caractérisé en ce que** n est un entier variant entre 1 et 2, de préférence n vaut 1.

5. Composé selon l'une quelconque des revendications 1 à 4 **caractérisé en ce que** ledit composé est une 2-(alkylthio)octan-4-one, une 2-(alkylsulfinyl)octan-4-one, une 2-(alkylsulfonyl)octan-4-one ou un alcanoate ou alcénoate de 4-oxooctan-2-yle, avec la taille du groupe alkyle ou alcényle variant entre C₆ et C₁₈.

6. Utilisation d'un composé de formule (I) selon l'une quelconque des revendications 1 à 5 en tant qu'ingrédient parfumant pour fournir une odeur de fraise longue durée à l'environnement.

7. Utilisation selon la revendication 6 **caractérisée en ce que** ledit composé de formule (I) est utilisé en combinaison avec au moins un ingrédient sélectionné parmi l'oct-2-én-4-one, la 2,5-diméthyl-4-hydroxy-2*H*-furan-3-one, la 5-éthyl-4-hydroxy-2-méthyl-3(2*H*)-furanone, l'acétate de 2,5-diméthyl-4-oxo-4,5-dihydrofuran-3-yle, le 3-méthyl-3-phényloxirane-2-carboxylate d'éthyle, le 3-phényloxirane-2-carboxylate d'éthyle, le 2-acétamidobenzoate de méthyle ou le 3-méthylbutanoate de 3-phénylpropyle, le propionate de 2-méthyl-4-oxo-4*H*-pyran-3-yle, l'acide 2-méthylpent-2-énoïque ou le (2S,5S)-2-(*tert-*butyl)-5-méthyl-2-propyltétrahydrofurane.

8. Procédé pour conférer, intensifier, améliorer ou modifier les propriétés d'odeur de fraise d'une composition parfumante ou d'un article parfumé, lequel procédé comprend l'ajout à ladite composition ou audit article d'une quantité efficace d'au moins un composé de formule (I) selon l'une quelconque des revendications 1 à 5.

9. Composition parfumante comprenant :
i) en tant qu'ingrédient parfumant, au moins un composé de formule (I) tel que défini dans l'une quelconque des revendications 1 à 5 ;
ii) au moins un ingrédient sélectionné dans le groupe constitué par un support de parfumerie et une base de parfumerie ; et
iii) facultativement, au moins un adjuvant d'usage courant en parfumerie.

10. Composition parfumante selon la revendication 9, **caractérisée en ce qu'**elle comprend en outre au moins un composé sélectionné parmi les isothiazolones de formule 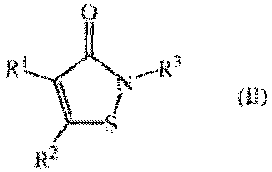
dans laquelle R¹ et R² représentent, séparément et indépendamment l'un de l'autre, un atome d'hydrogène, un atome d'halogène, de préférence le chlore, un groupe alkyle linéaire ou ramifié en C₁-C₄, un groupe amino ou un groupe benzylamino ; ou, en variante, R¹ et R² sont pris ensemble pour représenter un noyau phényle ou pyridine, éventuellement substitué par un à quatre groupes alkyle ou alcényle linéaires ou ramifiés en C₁-C₄ et/ou un à deux atomes d'halogène, de préférence des atomes de chlore ; et
R³ représente un atome d'hydrogène, un atome de métal alcalin, en particulier Na ou K, un groupe phényle ou benzyle éventuellement substitué par un ou deux atomes d'halogène et/ou un ou deux groupes méthyle, trifluorométhyle, méthoxy ou amino, un groupe amine, ou un groupe hydrocarboné insaturé en C₁-C₈ linéaire, ramifié ou cyclique éventuellement substitué par un ou deux atomes d'azote, d'oxygène ou d'halogène.

11. Produit de consommation parfumé qui comprend :
i) en tant qu'ingrédient parfumant, au moins un composé de formule (I), tel que défini dans l'une quelconque des revendications 1 à 5, ou une composition parfumante, telle que définie dans les revendications 9 ou 10 ; et
ii) une base de produit de consommation de parfumerie.

12. Produit de consommation parfumé selon la revendication 11, **caractérisé en ce que** le produit de consommation de parfumerie est un parfum, un produit d'entretien textile, un produit de soin corporel, un produit d'assainissement de l'air ou un produit d'entretien ménager.

13. Produit de consommation parfumé selon la revendication 11, **caractérisé en ce que** le produit de consommation de parfumerie est un parfum fin, un détergent liquide ou solide, un adoucissant pour textile, une eau de linge, une eau de repassage, un shampooing, une préparation colorante, une laque capillaire, un déodorant ou un anti-transpirant, un savon parfumé, un gel, une huile ou une mousse pour le bain ou la douche, un produit d'hygiène, un désodorisant d'air ambiant, un désodorisant d'air ambiant en poudre « prêt à l'emploi » ou un détergent pour surfaces dures.

14. Procédé pour conférer une odeur de fraise longue durée ou substantielle à des surfaces, telles que des surfaces dures, un textile, la peau ou les cheveux, par ajout d'au moins un composé de formule (I) selon les revendications 1 à 5 à des compositions parfumantes ou à des articles parfumés, et leur application sur la surface visée correspondante.
